# EUROPEAN PATENT SPECIFICATION

(11) **EP 0 264 797 B1**
(45) Date of publication and mention of the grant of the patent: **10.01.1996**
(21) Application number: 87114982.9
(22) Date of filing: 14.10.1987
(51) Int. Cl.: G01N 33/531, G01N 33/533, G01N 33/542

(54) **Benzodiazepines assay, tracers, immunogens and antibodies**
Test, Indikatoren, Immunogene und Antikörper für Benzodiazepine
Essai, traceurs, immunogènes et anticorps pour benzodiazépines

(30) Priority: 24.10.1986 US 922595
(43) Date of publication of application: 27.04.1988
(73) Proprietor: ABBOTT LABORATORIES, Abbott Park, Illinois 60064-3500 (US)
(72) Inventor: Wang, Nai-Yi, Mundelein, IL 60060 (US); Keegan, Candace Linda, Mundelein, IL 60060 (US); Heiman, Daniel Fuelner, Libertyville, IL 60048 (US); Flentge, Charles Arthur, Lake Villa, IL 60046 (US); Wang, Philip Pei, Libertyville, IL 60048 (US)
(74) Representative: Modiano, Guido, Dr.-Ing.

(56) References cited:
- EP-A- 0 218 010
- CH-A- 566 324
- FR-A- 2 110 275
- NL-A- 7 110 117
- US-A- 4 243 654
- US-A- 4 318 846

## Description

### 1. Technical Field

The present invention relates to a method and reagents for a fluorescence polarization immunoassay procedure for determining the presence or amount of benzodiazepines in fluids, especially biological fluids such as urine, serum or plasma, and to a method of making the reagents. More specifically, the invention relates to (1) tracers for determining the presence or amount of benzodiazepines and/or their metabolites in a sample; (2) synthetic methods for making the tracer compounds; (3) analytical methods for conducting the assay; and (4) a preferred antibody for use in the assay.

### 2. Background Art

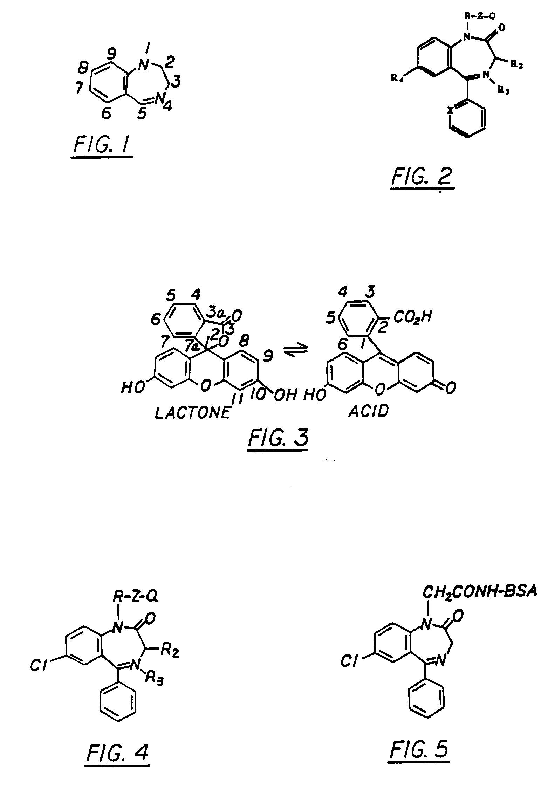

Benzodiazepine drugs constitute a class of compounds, most of which contain the benzodiazepine ring structure (Figure 1) bearing an aromatic substituent at the 5-position. They are used clinically as sedatives, hypnotics, muscle relaxants, anxiolytics, anti-epileptics and in the treatment of alcohol abuse. One benzodiazepine, diazepam, can cause a slight euphoric state and is also used illicitly either alone or as an adulterant in other illicit compounds.

Benzodiazepines are extensively metabolized, primarily by dealkylation at the 3-position in the case of 7-chloro-benzodiazepines. The 7-nitro-benzodiazepines metabolize to 7-amino- and 7-acetamido- forms as well. The hydroxylated metabolites are excreted primarily as glucuronide conjugates.

Few fatalities have been attributed to benzodiazepine ingestion alone. Benzodiazepines are generally considered to be safe compounds and this attitude has promoted their use. They are among the most frequently prescribed drugs in the Western world. The widespread use of benzodiazepines has led to accidental overdosage as well as intentional abuse, and analysis for their presence is typical during abused drug testing and in emergency rooms. Rapid, reliable, selective and accurate detection methods for benzodiazepines and their metabolites aid in this endeavor.

The biological fluid most frequently tested is urine. Urine samples are more accessible than blood samples, and other biological fluids have not been extensively investigated for use in assays.

In the past, the presence of benzodiazepines in urine has typically been detected by thin-layer chromatography (TLC) or enzyme immunoassay (EIA) with confirmation of levels in serum or plasma by high performance chromatography (HPLC) or gas chromatography (GC). These methods for analysis in urine are not without drawbacks. The TLC methods require sample extraction procedures and assay time is lengthy. EIAs involve enzyme reactions and have the following disadvantages: 1) the reagents are relatively unstable; 2) any components in the biological samples which may affect absorbance readings of the products of the enzyme reaction in an EIA (such as enzyme inhibitors or enzymes which catalyze similar reactions) will affect the assay results; and 3) EIAs measure absorbance, and any compounds in the biological samples (such as bilirubin or other chromophores) will affect the accuracy of the results obtained from these assays.

In assays for drugs and other substances, fluorescence polarization competitive binding immunoassays have provided a more satisfactory alternative. Typically, competitive binding immunoassays are used for measuring ligands in a test sample. (For the purposes of this disclosure, a "ligand" is a substance of biological interest to be determined quantitatively by a competitive binding immunoassay technique).

Benzodiazepine antigen conjugates and antibodies have been described in U.S. Patent No. 4,243,654 to Schneider et al., in U.S. Patent No. 4,083,948 to Davis et al., in U.S. Patent No. 4,191,738 to Dixon, and in U.S. Patent No. 4.046,636 to Ullman et al.

The present invention offers an advance in the art beyond that described supra, particularly in that highly sensitive fluorescent tracers, methods for preparing the fluorescent tracers, an assay employing such tracers and antibodies raised against a certain group of immunogens, and an antibody raised against a particularly effective immunogen are provided specifically for the determination of one or more benzodiazepines or benzodiazepine metabolites in a sample. An assay conducted in accordance with the present invention is particularly accurate, as will be explained infra.

As background art to the present invention, mention may be made of CH-A-566 324 which is directed to certain derivatives of benzodiazepines but does not disclose or suggest such compounds attached to fluorescent moieties or to immunogenic carriers.

Mention may also be made of FR-A-2 110 275 and GB-A-1 285 529 which are directed to certain derivatives of benzodiazepines for use as drugs. These references neither disclose nor suggest such compounds attached to fluorescent moieties or to immunogenic carriers.

### SUMMARY OF THE INVENTION

The present invention is directed to tracers for use in fluorescence polarization immunoassays for benzodiazepines and their metabolites; to methods for synthesizing the tracers; to methods for conducting such assays; and to a specific antibody.

A first aspect of the invention relates to the discovery of unique tracers having novel structures. These tracers can be represented by the general structural formula shown in Figure 2 of the drawings, wherein:
X is CH, N or C-halogen;
R₂ is -H or -OH;
R₃ is -O or a nonbonding electron pair;
R₄ is halogen, -NO₂, -NH₂ or -NHCOCH₃;
R is a linking group consisting of from 0 to 20 carbon atoms and heteroatoms, including not more than twelve heteroatoms, arranged in a straight or branched chain and containing up to two ring structures, with the proviso that not more than four heteroatoms may be linked in sequence, nor may more than two sulfur or nitrogen atoms or one oxygen atom be linked in sequence;
Z is C=O, C=NH, NH, NCH₃, -N=N-, SO₂ or CH₂; and
Q is an amino, amido, amidino, urea, thiourea, carbamate, thiocarbamate, triazinylamino, or (carboxyamino)-sulfonamido derivative of fluorescein or Q is 4-chloro-6-fluoresceinylamino-1,3,5-triazin-2-yl or 4-methoxy-6-fluoresceinylamino-1,3,5-triazin-2-yl.

Representative of the tracers of the invention are those wherein Q is a triazinylamino derivative of fluorescein.

Further representative are those tracers wherein Q is 4-chloro-6-(fluorescein-6-ylamino)-1,3,5-triazin-2-yl, fluorescein-5-yl carbonyl or fluorescein-6-yl carbonyl.

Still further representative are those tracers wherein Q is (fluorescein-5-ylamino)carbonyl or (fluorescein-6-ylamino)carbonyl.

Other representative tracers are those wherein Q is (fluorescein-5-yl)amino or (fluorescein-6-yl)amino.

A second aspect of the invention relates to methods for preparation of the novel tracers. According to this aspect of the invention, a tracer is made by chemically coupling a compound represented by the general structural formula shown in Figure 2 of the drawings wherein:
X is CH, N or C-halogen;
R₂ is -H or -OH;
R₃ is -O or a nonbonding electron pair;
R₄ is halogen, -NO₂, -NH₂ or -NHCOCH₃;
R is a linking group consisting of from 0 to 20 carbon atoms and heteroatoms, including not more than twelve heteroatoms, arranged in a straight or branched chain and containing up to two ring structures, with the proviso that not more than four heteroatoms may be linked in sequence, nor may more than two sulfur or nitrogen atoms or one oxygen atom be linked in sequence;
Z is NH, CO, SO₂ or -C=NH; and
Q is -H, -OH, or a leaving group with fluorescein or a derivative of fluorescein.

The synthesis of the tracers are discussed in section 3b.

Illustrative of the method of making the tracers is that wherein ZQ is NH₂ and the precursor is coupled by the steps of preparing an active ester of carboxyfluorescein and reacting the active ester with the precursor.

Further illustrative of the method of making the tracers is that wherein ZQ is CO₂H and the precursor is coupled by the steps of preparing an active ester of the precursor and reacting the active ester with amino-fluorescein.

Still further illustrative of the method of making the tracers is that wherein ZQ is NH₂ and the precursor is reacted with (4,6-dichloro-1,3,5-triazin-2-ylamino)-fluorescein or fluoresceinisothiocyanate.

Also illustrative of the method of making the tracers is that wherein a precursor is coupled by the steps of preparing an imidate ester of the precursor and reacting the imidate with aminofluorescein.

A further illustrative method of making the tracers is that wherein Q is OH and the precursor is coupled by reacting it with (4,6-dichloro-1,3,5-triazin-2-ylamino)fluorescein.

A third aspect of the invention relates to an analytical method for detecting the presence of benzodiazepines and benzodiazepine metabolites in a fluid sample which comprises the steps of:
(a) contacting said sample with antiserum raised against a benzodiazepine linked to an immunogenic carrier substance through its ring nitrogen adjacent to its ring carbonyl and with a tracer compound as described supra capable of producing a detectable fluorescence polarization response to the presence of said antiserum;
(b) passing plane polarized light through the resulting solution from step (a) to obtain a fluorescence polarization response; and
(c) detecting the fluorescence polarization response of the solution of step (b) as a measure of the presence of benzodiazepine metabolites in the sample.

In the analytical method of the invention, it is preferred that the antiserum is raised against an immunogen comprising the structure: 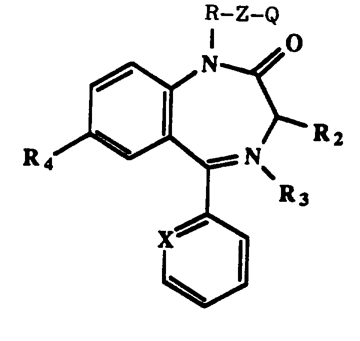 wherein:
X is CH, N or C-halogen;
R₂ is -H or -OH;
R₃ is -O or a nonbonding electron pair;
R₄ is halogen, -NO₂, -NH₂ or -NHCOCH₃;
R is a linking group consisting of from 0 to 20 carbon atoms and heteroatoms, including not more than twelve heteroatoms, arranged in a straight or branched chain and containing up to two ring structures, with the proviso that not more than four heteroatoms may be linked in sequence, nor may more than two sulfur or nitrogen atoms or one oxygen atom be linked in sequence;
Z is C=O, C=NH, NH, NCH₃, -N=N-, SO₂ or CH₂; and
Q is an immunogenic carrier substance.

The above immunogens can be prepared by chemically combining any compound represented by the structural formula shown in Figure 2 wherein:
X is CH, N or C-halogen;
R₂ is -H or -OH;
R₃ is -O or a nonbonding electron pair;
R₄ is halogen, -NO₂, -NH₂ or -NHCOCH₃;
R is a linking group consisting of from 0 to 20 carbon atoms and heteroatoms, including not more than twelve heteroatoms, arranged in a straight or branched chain and containing up to two ring structures, with the proviso that not more than four heteroatoms may be linked in sequence, nor may more than two sulfur or nitrogen atoms or one oxygen atom be linked in sequence;
Z is C=O, C=NH, SO₂, NH, NCH₃, or CH₂; and
Q is hydrogen, hydroxyl or a leaving group (with the proviso that when Z is CH₂, Q may not be hydrogen);

with a macromolecular or particulate carrier substance such as a poly(amino acid), a poly(amino acid) derivative or other macromolecular carrier or a synthetic polymeric bead bearing reactive functional groups on its surface.

For the purposes of this disclosure, a "leaving group" is a halogen, an acyloxy group (including carbonate ester), a N-succinimidyloxy or N-phthalimidyloxy group, an alkoxy or phenoxy or substituted phenoxy group, an N-imidazolyl group, a 1-benzotriazolyloxy group or any of the other similar activating groups well known to those skilled in the art.

Antibodies used in the analytical method of the invention are prepared in response to an immunogen employing in vivo or in vitro techniques and methods well known to those skilled in the art.

Another aspect of the invention relates to the elimination of potential fluorescence interferences by riboflavin or other fluorescent chromophores. Sodium iodide is added either directly to each sample or to one or more of the reagents utilized in the assay, wherein it quenches the fluorescence present, thus eliminating fluorescence interference.

Further objects and attendant advantages of the invention will be best understood from a reading of the following detailed description taken together with the Figures and the Examples.

### BRIEF DESCRIPTION OF THE DRAWINGS

In the following Figures the symbol "F1" represents fluorescein or a fluorescein derivative and the various other symbols are noted in the Detailed Description.

Figure 1 shows the basic structure of the class of benzodiazepines to be quantitatively or qualitatively determined in accordance with the present invention, including the numbering scheme used for these compounds.

Figure 2 shows a general structural formula for haptens, immunogens, tracers and tracer precursors.

Figure 3 shows the alternate structural formulae together with names and numbering schemes for the fluorescein moiety included in the tracers of the present invention.

Figure 4 shows a general structural formula for the preferred immunogens.

Figure 5 shows a structural formula for the most preferred immunogen.

Figure 6 shows a general structural formula for the preferred tracers of the present invention.

Figure 7 shows the various linkages that couple the fluorescein moiety to the tracer precursors of Figure 2.

Figures 8 through 12 show various examples of hapten reactants which can be used to form immunogens and tracers.

Figure 13 shows an immunogen which is included herein for purposes of comparison with the immunogens used in the method of the invention.

Figures 14 through 19 show various examples of structures of tracers in accordance with the present invention.

Figure 20 shows a tracer which is included herein for purposes of comparison with tracers of the invention.

Figures 21-22 show examples of synthetic intermediates employed in the synthesis of hapten and tracer precursors.

Figures 23-24 show examples of synthetic intermediates employed in the synthesis of hapten and tracer precursors which are included herein for illustrative purposes and which do not lead to immunogens and tracers used in the method of the invention.

### DETAILED DESCRIPTION OF THE INVENTION

The various aspects of the invention will now be discussed in detail in relation to the Figures.

The present invention involves the use of fluorescein and derivatives of fluorescein. In particular, a necessary property of fluorescein and its derivatives for the usefulness of the tracer compounds described herein is the fluorescence of fluorescein. Fluorescein exists in two tautomeric forms, illustrated in Figure 3, depending on the acid concentration (pH) of the environment. In the open (acid) form, there are a number of conjugated double bonds which make that form of fluorescein (and compounds containing a fluorescein moiety) capable of absorbing blue light and emitting green fluorescence after an excited state lifetime of about four nanoseconds. When the open and closed forms coexist, the relative concentration of molecules in the open and closed forms is easily altered by adjustment of the pH level. Generally, the tracer compounds of the present invention exist in solution as biologically acceptable salts such as sodium, potassium, ammonium and the like, which allows the compounds to exist in the open fluorescent form, when employed in the analytical methods of the present invention. The specific salt present will depend on the buffer employed to adjust the pH level. For example, in the presence of a sodium phosphate buffer, the compounds of the present invention will generally exist in the open form, as a sodium salt.

An used herein, the term "fluorescein," either as an individual compound or as a component of a larger compound, is meant to include both the open and closed forms, if they exist for a particular molecule, except in the context of fluorescence. An open form is necessary for the fluorescence to occur.

The numbering of carbon atoms of the fluorescein molecule varies, depending upon whether the open or closed form of the molecule is considered. Accordingly, the literature concerning fluorescein and its compounds is not uniform as to carbon atom numbering. In the closed form, the para-carbon to the carbonyl of the lactone on the phenyl ring is numbered "6." In the open form, the para-carbon to the carboxylic acid group on the phenyl ring is numbered "5" (see Figure 3). In this disclosure the numbering of the closed form is adopted because the raw materials used in the syntheses are most popularly numbered with that system. The carbon atom of fluorescein and its compounds which is opposite the carboxyl group is therefore numbered "6" for the purposes of the present disclosure.

A tracer in solution which is not complexed to an antibody is free to rotate in less than the time required for absorption and re-emission of fluorescent light. As a result, the re-emitted light is relatively randomly oriented so that the fluorescence polarization of a tracer not complexed to an antibody is low, approaching zero. Upon complexing with a specific antibody, the tracer-antibody complex thus formed assumes the rotation of the antibody molecule, which is slower than that of the relatively small tracer molecule, thereby increasing the polarization observed. Therefore, when a ligand competes with the tracer for antibody sites, the observed polarization of fluorescence of the resulting mixture of the free tracer and tracer-antibody complex assumes a value intermediate between that of the tracer and that of the tracer-antibody complex. If a sample contains a high concentration of the ligand, the observed polarization value is closer to that of the free ligand, i.e., low. If the test sample contains a low concentration of the ligand, the polarization value is closer to that of the bound ligand, i.e., high. By sequentially exciting the reaction mixture of an immunoassay with vertically and then horizontally polarized light and analyzing only the vertically polarized component of the emitted light, the polarization of fluorescence in the reaction mixture may be accurately determined. The precise relationship between polarization and concentration of the ligand to be determined is established by measuring the polarization values of calibrators with known concentrations. The concentration of the ligand can be interpolated from a standard curve prepared in this manner.

The particular tracers formed in accordance with this invention and the use of particular antibodies have been found to produce very good assays, as discussed infra.

### 1. The Reagents

Both the tracers of the present invention and the preferred immunogens used to raise antibodies for use in the assay of the present invention can be represented by the general structural formula set forth in the Summary of the Invention, and illustrated in Figure 2.

The objective is to have competition between benzodiazepines and the benzodiazepine metabolites and the tracer for the recognition sites of the antibody. Great variations in the structure of the haptens and tracers are allowed in achieving this goal. For the purpose of this invention, "haptens" are precursors of the immunogens, comprising generally a substituted benzodiazepine derivative bearing a group suitable for linking to an immunologically active carrier.

### a. The Structure of the Immunogens

Antibodies raised against immunogens functionalized at the 1-position are useful in benzodiazepines and benzodiazepine metabolites assay according to this invention when combined with the appropriate tracer.

The immunogens employed in raising antibodies for use in the assay of the present invention have the general structural formula shown in Figure 2 and, in a preferred form of the invention, the immunogens are derived from the general structural formula shown in Figure 4. The immunogens can be prepared by coupling a compound of the class shown in Figure 2 with a poly(amino acid) or a derivative of a poly(amino acid) or other immunologically active carrier as will be discussed in the context of the synthetic method and the Examples below.

The 1-position derivatives represented by the general structural formula of Figure 4 (wherein R, R₂ and R₃ are defined as supra, Z is C=O, C=NH, SO₂, NH, NCH₃ or CH₂ and Q is an immunogenic carrier) are the preferred embodiment of the immunogens to be used in the method of the invention.

In the most preferred form of this aspect of the invention, the immunogens are represented by the structural formula of Figure 5. This structure is preferred because it provides antisera with sensitivity to a broad range of benzodiazepines and benzodiazepine metabolites while excluding other drugs and endogenous substances. Although bovine serum albumin is the poly(amino acid) in this most preferred form, it should be understood that various protein carriers can be employed, including albumins, serum proteins, e.g., globulins, ocular lens proteins, lipoproteins and the like. Illustrative protein carriers include bovine serum albumin, keyhole limpet hemocyanin, egg ovalbumin, bovine gamma globulin, thyroxine binding globulin, etc. Alternatively, synthetic poly(amino acids) having a sufficient number of available amino groups such as those on lysine or ornithine residues can be employed, as can many other synthetic or natural polymeric materials bearing reactive functional groups. In addition, carbohydrates, yeasts, polysaccharides or any other substance that can be used as an immunological carrier can be conjugated to the hapten to produce an immunogen.

### b. The Structure of the Tracers

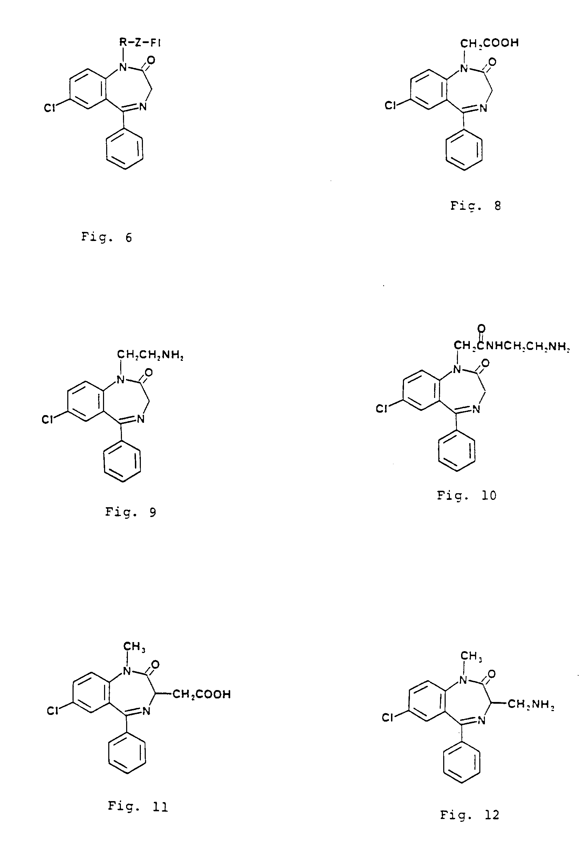

The possible variations in the structure of the tracers of the present invention are even greater than the possible variations in the structure of the haptens. The tracers have the general structural formula shown in Figure 2. In a preferred form of the invention, the tracers have the structural formula shown in Figure 6, wherein R is as defined supra and Z is C=O, NH, SO₂ or C=NH.

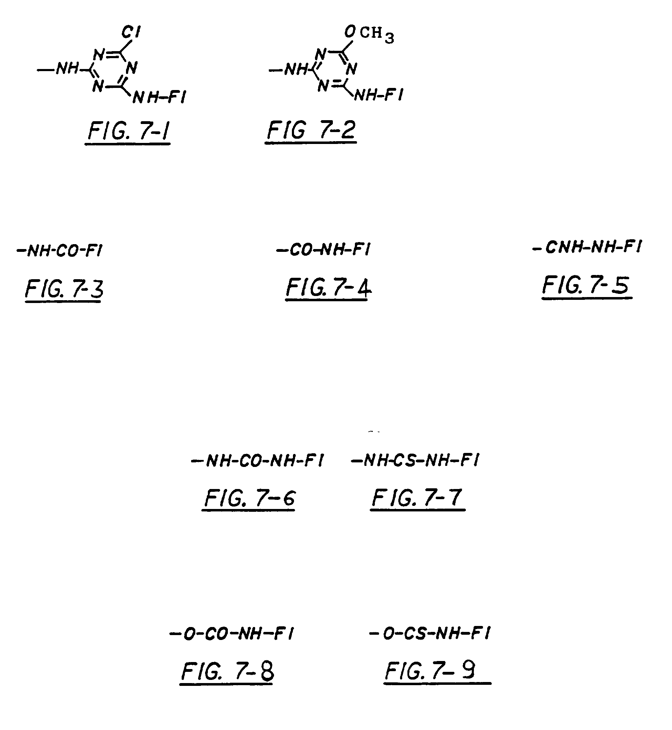

The tracer is a benzodiazepine derivative that is linked to a fluorescein derivative by, for example, an amido, amidino, triazinylamino, carbamido, thiocarbamido, carbamoyl, thiocarbamoyl, or sulfonylcarbamoyl group, as shown in Figure 7. The tracers are prepared by linking the appropriate fluorescein derivative to a benzodiazepine derivative containing an amino, carboxylic acid, sulfonic acid, mercapto, hydroxy, imidate, hydrazide, isocyanate, thioisocyanate, chloroformate, chlorothioformate, chlorosulfonylcarbamoyl, or similar group, as will be discussed in the context of the synthetic method and the Examples below.

By way of example, any of the following fluorescein derivatives can be used as reactants:

| | |
|---|---|
| F1-NH₂ | fluorescein amine |
| F1-CO₂H | carboxyfluorescein |
| F1-NHCOCH₂I | -iodacetamidofluorescein |
| F1-NHCOCH₂Br | -bromoacetamidofluorescein 2,4-dichloro-1,3,5-triazin-2-ylaminofluorescein (DTAF) 4-chloro-6-methoxy-1,3,5-triazin-2-ylaminofluorescein |
| F1-NCS | fluorescein thioisocyanate |
| F1-CH₂NH₂ | 11-aminomethyl fluorescein |

### 2. The antibodies

The antibodies employed in the assay of the present invention are prepared by eliciting a response in sheep or rabbits to the immunogens described supra. The immunogen is administered to animals or to in vitro cultures of immunocompetent cells by a series of inoculations, in a manner well known to those skilled in the art.

### 3. Synthetic Methods

Both the tracers of the present invention and the immunogens used to raise antibodies for use in the assay of the present invention can be made from a precursor having the general structural formula shown in Figure 2.

### a. The Synthesis of the Immunogens

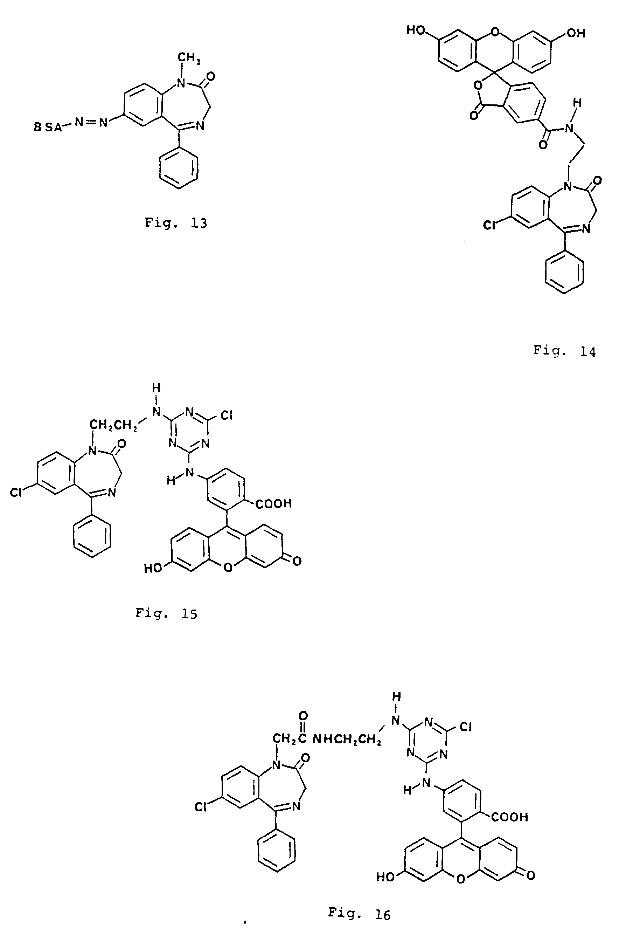

The immunogens are made by coupling a hapten, such as that shown by the general structure of Figure 2, to a poly(amino acid). The poly(amino acid) moiety can be linked to the hapten by an amide, an amidine, an alkyl, a urea, a thiourea, a carbamate, or a thiocarbamate linkage. In a preferred embodiment, the poly(amino acid) is bovine serum albumin (BSA) and the hapten is shown in Figure 8. The hapten is preferably coupled under conditions normally used to form amide linkages, which conditions are well known to those skilled in the art. The immunogens are prepared by coupling a hapten that contains an -NH₂, -CO₂H, -CONHNH₂, -CNOR, -CHO, -Br, -I, -NCO, -NCS, -OCOCl -SO₂Cl or -OCSCl group to a poly(amino acid). Haptens containing an NH₂ group can be coupled by activating the carboxylic acid group on the poly(amino acid) in the presence of the -NH₂ group. For aromatic amines, the diazonium salt method can be used. The diazonium salt, prepared by mixing the amine with sodium nitrite in acid solution, is added to the poly(amino acid). Activation of the carboxylic acid groups on the poly(amino acid) can be accomplished by mixing the hapten and the poly(amino acid) with 1-ethyl-3-(3-dimethylaminopropyl) carbodiimide (EDC), N,N'-dicyclohexylcarbodiimide (DCC), 1-cyclohexyl-3-(2-morpholinoethyl)carbodiimide methoxy-p-toluenesulfonate, or the like. Carboxylic acid-containing haptens are also coupled by the in situ activation method (EDC) or the active ester method, as described below in the tracer synthesis section. For -CONHNH₂, coupling is performed as for a non-aromatic amino group. A -CNOR compound, which is prepared from the corresponding cyano compound, is coupled directly to the poly(amino acid). A -CHO compound is coupled to the poly(amino acid) by reductive amination. The poly(amino acid) is mixed with the -CHO hapten and the resulting imine is reduced with a borohydride reducing agent such as sodium cyanoborohydride to yield alkylated amines on the poly(amino acid). Isocyanate (-NCO) and isothiocyanate (-NSC) compounds, which are prepared from the corresponding amino compound, and chloroformate (-OCOCl) and chlorothioformate (-OCSCl) compounds, which are prepared from the corresponding alcohol compound, produce urea, thiourea, carbamate and thiocarbamate linkages, respectively. This is accomplished by directly coupling the hapten to the poly(amino acid).

The syntheses of the above haptens (immunogen precursors) are accomplished in very similar ways. Figure 4 shows an immunogen precursor class in accordance with a preferred embodiment.

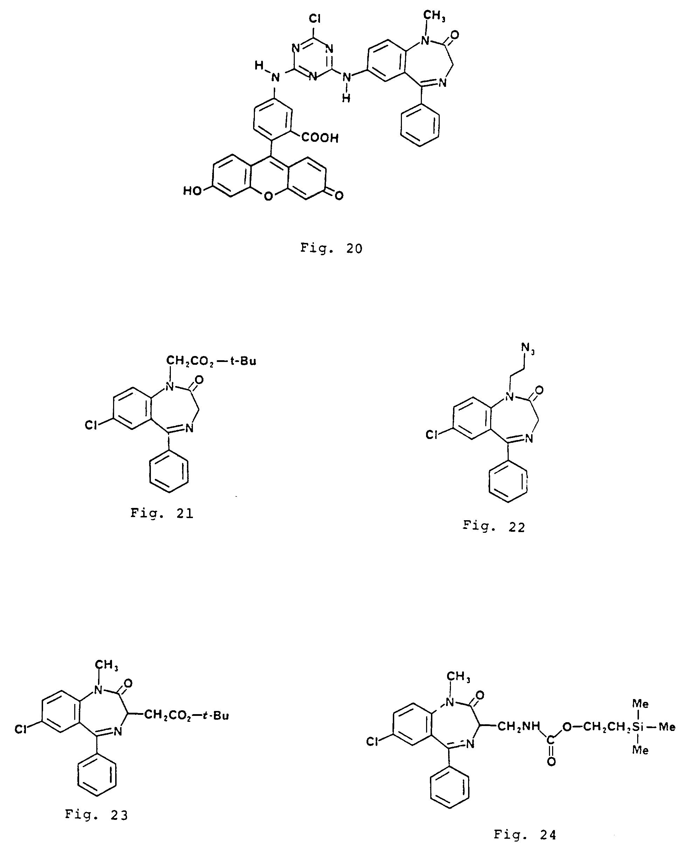

In general, the 1-substituted haptens are prepared by alkylation of the amide nitrogen of nordiazepam or its derivatives with an ester of an omega-halocarboxylic acid, an omega-haloazide or a sulfonate ester of an omega-azido primary alcohol. The latent or protected functionality is then revealed and the compound is coupled to the poly(amino acid) or other carrier. For example, in the case of the t-butyl ester protected side chain (Figure 21), the protecting group was removed by treating with trifluoroacetic acid, while in the case of the azide-terminated chain, the latent amine functionality was revealed by selective reduction with propane dithiol or triphenylphosphine. It is possible to alkylate nordiazepam or its derivatives with other bifunctional alkylating agents such as bromoacetonitrile, bromoacetaldehyde dimethylacetal and the like.

Aldehydes or ketones can be derivatized by known methods to a variety of compounds containing a suitable group useful for coupling to a carrier protein, such as, Wittig reaction, condensation with hydrazine compounds, reductive amination with amino compounds or the like.

Aldehydes and ketones can also be condensed with (aminohydroxy)alkylcarboxylic acids, such as NH₂OCH₂CO₂H, to produce substituted oxime derivatives. The oxime alkyl carboxylic acid derivatives can be partially reduced to the corresponding (aminohydroxy)alkylcarboxylic acid derivatives. The same type of condensation and reduction can be accomplished with hydrazine and hydrazine derivatives.

Nitrile derivatives can be converted to alkoxy imidates by treating the nitrile with anhydrous alcohol and hydrogen chloride gas. The hydrazide derivatives can be prepared from the corresponding carboxylic acid derivatives by active ester coupling with hydrazine or by reacting hydrazine with the corresponding carboxylic ester derivative. Amines are convertible to the isocyanate or thioisocyanate derivatives and alcohols are convertible to chloroformate and chlorothioformate derivatives by reaction of the amine or the alcohol with phosgene or thiophosgene.

### b. The Synthesis of the Tracers

The tracers of the present invention are made by coupling a fluorescein moiety, or a derivative of fluorescein, to the general structure shown in Figure 2. The fluorescein moiety can be linked to the amino, carboxyl, imidate or alkoxy functional group by an amide, an amidine, a urea, a thiourea, a carbamate, a thiocarbamate, triazinylamino or sulfonylcarbamate linkage, as shown in Figure 7. In the presently preferred embodiment, the fluorescein derivative is 6-(4,6-dichloro-1,3,5-triazin-2-yl) amino)fluorescein, and this is coupled to precursors shown in Figures 9 and 10.

All benzodiazepine derivatives that have a terminal amino group, such as amino, hydrazinyl, hydrazido or the like, are coupled to carboxyfluorescein by the active ester method or the mixed anhydride method, and coupled to fluorescein isothiocyanate, DTAF or alkoxy DTAF by simply mixing the two materials in solution. The amino group can be converted to the isocyanate and thioisocyanate groups by reaction with phosgene and thiophosgene, respectively. These are then condensed with aminofluorescein to produce the tracer.

All benzodiazepine derivatives that have a terminal carboxylic acid group, such as carboxylic acid, (aminohydroxy)alkylcarboxylic acid or the like, are coupled to aminofluorescein and aminomethylfluorescein by the active ester method.

Any benzodiazepine derivatives having a terminal hydroxy group can be coupled to fluorescein by reaction with DTAF, -bromoacetamidofluorescein or fluorescein isothiocyanate in solution. The hydroxy group can be converted to the chlorosulfonylcarbamoyl, chloroformate or chlorothioformate groups by reaction with chlorosulfonylisocyanate, phosgene or thiophosgene, respectively. These derivatives are then coupled to aminofluorescein in solution to produce the tracers.

Benzodiazepine derivatives having a terminal mercapto group are prepared from halides or sulfonate esters and alkali metal sulfides as described supra for azides. They are coupled in solution with -bromo- acetamidofluorescein or -bromoacetamidofluorescein.

Benzodiazepine derivatives that have a terminal nitrile group can be prepared from halides or sulfonate esters as described, supra, for azides. They are converted to imidates in anhydrous alcohol in the presence of hydrogen chloride gas. The imidate is then coupled to fluorescein amine in solution to prepare the tracer.

Preparation of the various amino, hydroxy and mercapto derivatives of the benzodiazepine derivatives has been described above in the immunogen preparation sections.

### 3. The Assay

The particular tracers and antibodies of the present invention have been found to produce surprisingly good results in fluorescence polarization assays for benzodiazepines and benzodiazepine metabolites that can be qualitatively or quantitatively determined in accordance with the present invention. The assay of the present invention provides a more rapid benzodiazepines and benzodiazepine metabolites assay method than most prior art methods, because it requires no specimen treatment before analysis. The assay system accurately detects the presence of benzodiazepines and benzodiazepine metabolites in a sample.

In accordance with the analytical methods of the present invention, i.e., the methods of determining benzodiazepines and benzodiazepine metabolites by a fluorescence polarization immunoassay procedure using the above-described tracer compounds and immunogens, a sample containing or suspected of containing benzodiazepines or benzodiazepine metabolites is intermixed with a biologically acceptable salt of a tracer and an antibody specific to benzodiazepines and benzodiazepine metabolites and the tracer. The antibody is produced using the immunogen described above. The benzodiazepines and benzodiazepine metabolites and tracer compete for limited antibody sites, resulting in the formation of complexes. By maintaining constant the concentration of tracer and antibody, the ratio of benzodiazepines- and benzodiazepine metabolites-antibody complex to tracer-antibody complex that is formed is directly proportional to the amount of benzodiazepines and benzodiazepine metabolites in the sample. Therefore, upon exciting the mixture with linearly polarized light and measuring the polarization of the fluorescence emitted by a tracer and a tracer-antibody complex, one is able to determine qualitatively whether or not benzodiazepines and benzodiazepine metabolites are present in the sample.

The results can be quantified in terms of net polarization units and span (in millipolarizaton units). The measurement of millipolarization units indicates the maximum polarization when a maximum amount of the tracer is bound to the antibody, in the absence of any benzodiazepines and benzodiazepine metabolites. The higher the net millipolarization units, the better the binding of the tracer(s) to the antibody. The span is an indication of the difference between the net millipolarization and the amount of tracer bound to the antibody at the minimum concentration above which the sample is defined as containing benzodiazepines and benzodiazepine metabolites. A larger span provides for a better numerical analysis of data. The preferred antibody-tracer(s) combination has a span of at least 30 millipolarization units, but a span of at least 5 millipolarization units at this minimum detectable concentration is acceptable.

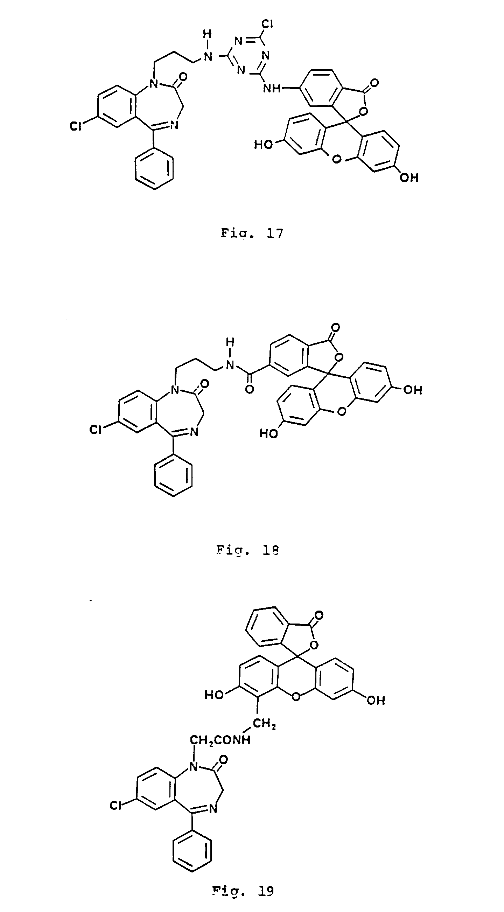

Table I shows the results obtained with various embodiments of the present invention, in terms of span and net millipolarization units. In all instances, bovine serum albumin was used as the protein carrier. As seen from the data in Table I, an assay produced from an immunogen made from the hapten of Figure 5 used in combination with tracers of Figures 17 and 19 provides excellent results. Accordingly, this combination is the most preferred form of the invention. In addition the hapten/tracer(s) combination of Fig. 15/Fig. 16, Fig. 15/Fig. 17, and Fig. 15/Fig. 19 also produced acceptable results and are alternative preferred combinations. The hapten/tracer combination of Fig. 13/Fig. 20 does not form part of the present invention and is shown in Table I for purposes of comparison with the hapten/tracer combinations of the invention.

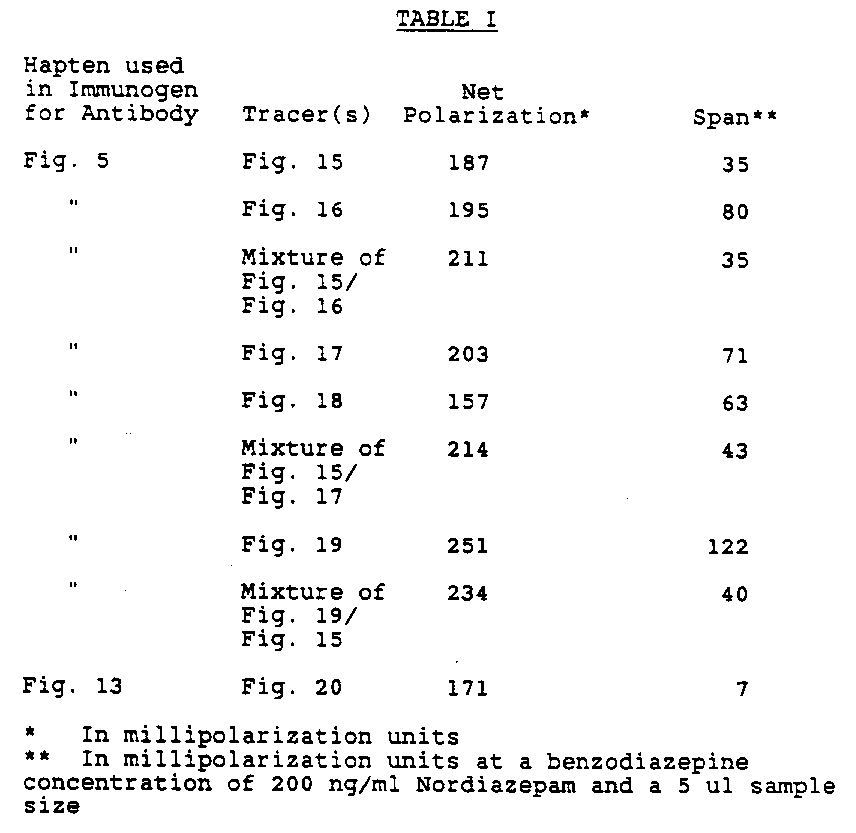

Sodium iodide (NaI) may be added to the sample or to one or more of the assay reagents other than the tracer in order to quench any fluorescence inherent in the sample, thus eliminating potential fluorescence interference in the assay. The amount of NaI employed is not critical, provided that a sufficient quantity is used to quench fluorescence by about fifty percent in the sample.

Additionally, largely conventional solutions including a pretreatment solution, a dilution buffer, benzodiazepines calibrator and benzodiazepines controls are desirably prepared. Typical solutions of these reagents, some of which are described below are commercially available in assay "kits" from Abbott Laboratories, Abbott Park, Illinois.

All percentages expressed herein are in weight/volume unless otherwise indicated. The tracer formulation presently preferred is 120 to 240 nanomolar tracer in 0.1 molar phosphate buffer at pH 7.5; 0.2% Polysorbate 80; 0.1% sodium azide; and 0.01% bovine gamma-globulin. The antisera formulation comprises sheep serum diluted with 0.1 molar tris buffer at pH 7.5; 0.1% sodium azide; 0.01% bovine gamma-globulin; and 2.0% ethylene glycol (volume/volume). The dilution buffer comprises 0.1 molar sodium phosphate at pH 7.5; 0.1% sodium azide; and 0.01% bovine gamma-globulin. The pretreatment solution comprises 50% sodium iodide; 0.9% sodium chloride; and distilled water. Benzodiazepine calibrators comprising nordiazepam in normal human urine at concentrations of 0.0, 200.0, 400.0, 800.0, 1200.0, and 2400.0 nanograms per milliliter with 0.1% sodium azide as a preservative are useful. Benzodiazepine controls comprised of nordiazepam in normal human urine at concentrations of 300.0 and 1000.0 nanograms per milliliter with 0.1% sodium azide as a preservative are also useful.

The fluorescence polarization value of each calibrator, control or samples is determined and is printed on the output tape of an instrument such as the Abbott TDx^{R} polarization analyzer. A standard curve is generated in the instrument by correlating the polarization of each calibrator versus its concentration using a nonlinear regression analysis. The concentration of each control or sample is read off the stored calibration curve and printed on the output tape. With respect to the foregoing preferred procedure, it should be noted that the tracer, antibody, pretreatment solution, calibrators and controls should be stored between about 2 and about 8 degrees C, while the dilution buffer should be stored at ambient temperature. A standard curve and controls should be run every two weeks, with each calibrator and control run in duplicate. Controls should be run daily and all samples can be run in replicates if so desired.

It should be understood that the foregoing detailed description and the following Examples are intended to be illustrative with respect to the scope of the present invention. Various modifications will become apparent to one skilled in the art, and thus it is intended that the scope of the invention be defined solely by the claims and legal equivalents thereof.

### EXAMPLES

Examples I through XXVII describe experiments that were performed in accordance with the concepts of the present invention. Example I and II are directed to preparations of immunogens useful for producing antibodies; Examples III through XIV are directed to the synthesis of precursors for immunogens and tracers; and Examples XV through XXVII are directed to the preparation of tracers.

### Example I

### Preparation of the Immunogen of Fig. 5

1-Carboxymethyl-7-chloro-1,3-dihydro-5-phenyl-2H-1,4-benzodiazepin-2-one (56 mg) was combined with dicylcohexylcarbodiimide (60 mg) and N-hydroxy succinimide (32 mg) and pyridine (1 ml). The mixture was stirred for three hours. The mixture was added dropwise to 10 ml of a solution comprising of bovine serum albumin (10 mg/ml) in dimethylformamide (25%). This solution was then stirred at room temperature for 18 hours, after which it was filtered through glass wool to remove precipitate from reaction. The mixture was dialyzed in a cellulose dialysing tube (Spectra/Por 3, M.W. cutoff 3500) against 25% dimethylformamide for two days followed by dialysis against distilled water for three days. The solution from the dialysis tubing was found to contain 8.6 mg/ml protein via the Biuret protein concentration determining method.

### Example II

### Preparation of the Immunogen of Figure 13

The immunogen of Figure 13 was prepared in connection with the comparative data of Table I.

50 mg (0.19 mmol) of 1-methyl-7-amino-1, 3-dihydro-5-phenyl-2H-1,4-benzodiazepin-2-one was dissolved in 5.0 ml of N-Dimethylformamide (DMF), 1.5 ml of water and 0.5 ml of 1M hydrochloric acid. The mixture was cooled to 4°C and 0.15 ml of 1M sodium nitrite (0.15 mmol) was added to the above mixture. The reaction mixture was stirred at 4°C for 30 minutes. 125 ul of 1M urea in water was added to the mixture to quench the reaction. The resulting solution was added dropwise to a solution of bovine serum albumin (500 mg) in 12.5 ml of sodium borate buffer (0.1M, pH 9.0) and was stirred for one hour at 4°C (pH was maintained at 9.0 with 1 N.NaOH).

This reaction mixture was maintained for 16 hours at 4°C with mixing. The resulting solution was dialyzed, first against 4 liters of sodium bicarbonate (0.05M) with 3 changes of the buffer. This was followed by dialyzing against 4 liters of deionized water (four changes). The resulting solution was the immunogen that would be injected into experimental animals.

### Example III

### 1-[tert-Butoxycarbonylmethyl]-7-chloro-1,3-dihydro-5-phenyl-2H-1,4-benzodiazepin--2-one (Fig. 21)

To a solution of 7-chloro-1,3-dihydro-5-phenyl-2H-1,4-benzodiazepin-2-one (264 mg, 9.7 mmol) in 20 ml of anhydrous N,N-dimethylformamide (DMF) under nitrogen was added sodium methoxide (659 mg, 12.8 mmol). The mixture was headed in an oil bath at 85°C for 30 minutes with stirring, and then a solution of tert-butyl bromoacetate (1.9 g, 9.7 mmol) in 1 ml of DMF was added over 10 minutes. After stirring was continued at the same temperature for 4 hrs, the mixture was concentrated in vacuo and partitioned between 90 ml of methylene chloride and 180 ml of water. The aqueous layer was extracted with two more portions of methylene chloride and the combined organic layers were washed with 45 ml of brine and dried (MgSO₄). After rotary evaporation the crude product was further dried in vacuo and purified by recrystallization from hexane/ethyl acetate (1:1) to give 1.99 g (53%) of a white powder.

### _{Example IV}

### 1-Carboxymethyl-7-chloro-1,3-dihydro-5-phenyl-2H-1,4-benzodiazepin-2-one (Fig. 8)

To a solution of 1-[tert-butoxycarbonylmethyl]-7-chloro-1,3-dihydro-5-phenyl-2H-1,4-benzodiazepin-2-one (275 mg, 0.71 mmol) in 5 ml of methylene chloride was added 2 ml of trifluoroacetic acid. After stirring at room temperature for 4 hours, the volatiles were removed in vacuo, and the residue was flash-chromatographed over silica gel (EM 9385). Elution with hexane/ethyl acetate (1:5) gave 240 mg of a light yellow glass.

### Example V

### 1-[(2-Aminoethylamine)carbonyl]methyl-7-chloro-1,3-dihydro-5-phenyl-2H-1,4-benzodiazepin-2-one (Fig. 10)

A mixture of 1-(carboxymethyl)-7-chloro-1,3-dihydro-5-phenyl-2H-1,4-benzodiazepin-2-one (35 mg mmol), dicyclohexylcarbodiimide (41 mg, 0.2 mmol), N-hydroxysuccinimide (13 mg, 0.11 mmol), and pyridine (0.25 ml) was stirred at room temperature for one hour. After the addition of ethylenediamine (30 mg, 0.5 mmol), the mixture was stirred for 5 additional hours. Water was then added and the aqueous mixture was extracted with ethyl acetate (three times). The combined extracts were washed with brine and dried over magnesium sulfate. Rotary evaporation of the solution gave a crude product which was recrystallized from ethyl acetate to give a colorless powder (24 mg) which had no aromatic proton in the NMR spectrum. The mother liquor was applied onto a preparative TLC plate (20 cm x 230 cm x 0.5 mm). Development with methanol-ammonium hydroxide (99:1) yielded 6.4 mg of ninhydrin positive (also UV active) material which gave the correct NMR spectrum.

### General Procedure for Preparing DTAF Tracers (G1)

A mixture of the amine (0.01 mmol), DTAF(I or II) (0.01 mmol), triethylamine (2 drops) and methanol (0.1 ml) was stirred at room temperature for 16 hours. The mixture was applied onto a preparative silica gel TLC plate. Development with CHCl₃/MeOH (3:1 or 4:1) gave fluorescent bands which were scraped off the plate and eluted with methanol separately. In selected cases, the relatively pure tracer was further purified on a reverse-phase preparative TLC plate (Whatman 4803--800, KC-18 F254) using acetonitrile/0.01M phosphate buffer (pH 5.3), (1:1, v/v) as developer.

### General Procedure for Preparing Carboxylfluorescein Tracers (GII)

A mixture of the amine (0.01 mmol), fluorescein carboxylic acid (V or VI)-O-succinimide ester (0.01 mmol) and pyridine (0.1 ml) was stirred at room temperature for 16 hours. The mixture was applied to a preparative TLC plate. Development with CHCl₃/MeOH (3:1 or 4:1) gave fluorescent bands which were scraped off the plate and eluted with methanol separately.

### General Procedure for Preparing Fluoresceinamine Tracers (GIII)

A mixture of the carboxylic acid (0.01 mmol), dicyclohexylcarbodiimide (0.02 mmol) and N-hydroxysuccinimide (0.012 mol) in dry pyridine (0.1 ml) was stirred at room temperature for 1 hour. The active ester formed was then treated with fluoresceinamine (isomer I or II) at the same temperature for 16 hours. The reaction mixture was applied to a preparative silica gel tlc plate (20 cm x 20 cm x 0.5 mm). Development with CHCl₃/MeOH (3:1 or 4:1 depending on the polarity of the substrate) gave fluorescent bands which were scraped off the plate. The individual bands were eluted with methanol and the eluents were collected.

### Example VI

### 1-(2-Azidoethyl)-7-chloro-1,3-dihydro-5-phenyl-2H-1,4-benzodiazepin-2-one (Fig. 22)

To a solution of 7-chloro-1,3-dihydro-5-phenyl-2H-1,4-benzodiazepin-2-one (270 mg, 1 mmol) in 3 ml of anhydrous N,N-dimethylformamide (DMF) under nitrogen was added sodium methoxide (freshly prepared from 30 mg of sodium and 3 ml of methanol). The mixture was heated in an oil bath at 85°C for 15 minutes with stirring and then a solution of 2-azidoethyl mesylate in DMF (260 mg) was added. After stirring was continued for 8 hours, the mixture was concentrated and partitioned between water and methylene chloride (three times). The combined organic phases were washed with H₂O and brine (once each). After drying over magnesium sulfate, the solution was rotary evaporated to give a crude product (335 mg).

### Example VII

### 1-Aminoethyl--7-chloro-1,3-dihydro-5-phenyl-2H-1,4-benzodiazepin-2-one (Fig. 9)

A mixture of the crude azide (310 mg) prepared in Example VI, propane dithiol (1.1 ml), and triethylamine (0.9 ml) in 30 ml of methanol was stirred at room temperature overnight. The mixture was diluted with water and extracted with ethyl acetate (three times). The extracts were combined, washed with brine, and dried (magnesium sulfate). Rotary evaporation of the solution gave a crude residue which was flash-chromatographed over silica gel (120 ml). Elution with methanol-NH₄OH (700:1) gave a ninhydrin positive and UV active material (109 mg) which was characterized by high-field (360 MHZ) proton NMR spectrum.

### Example VIII

### 1-(3-Azidopropyl)-7-chloro-5-phenyl-2H-1,4-benzodiazepin-2-one

Solvent was removed from 0.375 ml of 1.0 M potassium ethoxide in ethanol. Dimethylformamide (1.0 ml) and 7-chloro-1,3-dihydro-5-phenyl-2H-1, 4-benzodiazepin-2-one (81 mg) were added, and the mixture was heated in an oil bath at 80°C for 15 minutes. After cooling to room temperature, 81 mg of 3-azidopropyl methanesulfonate in 0.15 ml of dimethylformamide was added and the mixture was again heated in the 80°oil bath for one hour, when it had solidified to a gel. Partitioning between dichloromethane and water, washing with brine, drying with magnesium sulfate, filtration and removal of solvents gave the crude product, which was purified by chromatography on a thick-layer silica gel plate developed with hexane-ethyl acetate to give the title compound.

### Example IX

### 1-(3-Aminopropyl)-7-chloro-1,3-dihydro-5-phenyl-2H-1,4-benzodiazepin-2-one

A solution of 40 mg of 1-(3-azidopropyl)-7-chloro-1,3-dihydro-5-phenyl-2H-1,4-benzodiazepin-2-one and 35 mg of triphenylphosphine in 2 1/2 ml of anhydrous THF was stirred at room temperature for 24 hours. Water (0.0026 ml) was added and stirring was continued for 3 hours more. Removal of volatile materials left a mixture of the title compound and triphenylphosphine oxide, which was used to prepare tracers without further purification.

### Example X

### 7-Chloro-1-(fluorescein-11-ylmethylaminocarbonylmethyl)-5-phenyl-1,3-dihydro-2H-1,4-benzodiazepin-2-one (Fig. 19)

40 Milligrams of the acid prepared as described in Example IV was taken up in 2 ml of N,N-dimethyl formamide and N-hydroxysuccinimide (15 mg) was added followed by 1,3-dicyclohexylcarbodiimide (28 mg). Stirring was continued at room temperature for 16 hours. This was filtered into a solution of aminomethylfluorescein (48 mg) and triethylamine (24 mg) in 3 ml of N,N-dimethylformamide. The mixture was stirred at room temperature for 24 hours.

### Purification

- FIRST -: Whatman C18, 20 x 20 cm,/mm prep plates solvent system: 70/30/.5 methanol, water, acetic acid R_{f} = 0.2
- SECOND: Silica gel, 20 x 20 cm,/mm prep plate solvent system: 90/10 methylene chloride-methanol R_{f} = 0.65

### Example XI

### 3-[(t-Butoxycarbonyl)methyl]-7-chloro-1,3-dihydro-1-methyl-5-phenyl-2H-1,4-benzodiazepin-2-one (Fig. 23)

This example is given for illustrative purposes and does not produce a precursor useful for making immunogens or tracers to be used in the method of the invention.

Prepared from diazepam by a procedure similar to that described in Example III.

### Example XII

### 3-(carboxymethyl)-7-chloro-1,3-dihydro-1-methyl-5-phenyl-2H-1,4-benzodiazepin-2-one (Fig. 11)

For the procedure of removing the tert-butoxy group see Example IV.

### Example XIII

### F-Chloro-1,3-dihydro-1-methyl-5-phenyl-3-[(2-trimethylsilylethoxy)carbonylaminomethyl]-2H-1,4-benzodiazepin-2-one (Fig. 24)

This example is given for illustrative purposes and does not produce a precursor useful for making immunogens or tracers to be used in the method of the invention.

A mixture of the carboxylic acid (prepared in Example XII, 85 mg, 0.248 mmol), diphenylphosphoryl azide (68 mg, 0.248 mmol), triethylamine (25 mg, 0.248 mmol) and toluene (1.5 ml) was stirred at 80°C for 2h under nitrogen. 2-(Trimethylsilyl)ethanol (59 mg, 0.499 mmol) was added and the resulting mixture was heated at the same temperature for additional 6 hours with stirring.

After cooling to room temperature, the mixture was partitioned between water and ethyl acetate. The combined organic layers were washed with brine (once) and dried over magnesium sulfate. Rotary-evaporation of the solution gave 171 mg of oil. One third of the sample was applied to a preparative TLC plate (silica gel, 20 cm x 20 cm x 2 mm). Development with ethyl acetate/hexane (5:1) gave a major band (R_{f}= 0.52) which was scraped off the plate. Eluting with methanol gave 26 mg of an oil.

### Example XIV

### 3-Aminomethyl-7-chloro-1,3-dihydro-1-methyl-5-phenyl-2H-1,4-benzodiazepin-Z-one (Fig. 12)

To a solution of the carbamate (prepared according to Example XIII, 24 mg, 0.052 mmol) in THF (1 ml) was added via a syringe 0.21 ml of 1M (n-Bu)₄NF in THF. The resulting solution was stirred at 50°C for 30 minutes. Ethyl acetate was added and the diluted mixture was washed with ammonium chloride solution and water (once each). After drying over magnesium sulfate, the solution was rotary evaporated to afford 25 mg of an oil.

### Example XV

### 1-(2-[4-chloro-2-(fluorescein-5-ylamino)-1,3,5-triazin-6-ylamino]ethyl)-7-chloro-1,3-dihydro-5-phenyl-2H-1,4-benzodiazepin-2-one.

Prepared according to the general procedure (G1).

### Example XVI

### 1-(2-[4-chloro-2-(fluorescein-6-ylamino)-1,3,5-triazin-6-ylamino]ethyl)-7-chloro-1,3-dihydro-5-phenyl-2H-1,4-benzodiazepin-2-one (Fig. 15)

Prepared according to the general procedure (G1).

### Example XVII

### 1-[2-(fluorescein-5-ylcarbonyl)aminoethyl]-7-chloro-1,3-dihydro-5-phenyl-2H-1,4-benzodiazepin-2-one (Fig. 14)

Prepared according to the general procedure (GII).

### Example XVIII

### 1-[2-(fluorescein-6-ylcarbonylamino)ethyl]-7-chloro-1,3-dihydro-5-phenyl-2H-1,4-benzodiazepin-2-one

Prepared according to the general procedure (GII).

### Example XIX

### 1-[(fluorescein-5-ylamino)carbonyl methyl]-7-chloro-1,3-dihydro-5-phenyl-2H-1,4-benzodiazepin-2-one

Prepared according to the general procedure (GIII).

### Example XX

### 1-(fluorescein-6-ylaminocarbonylmethyl)-7-chloro-1,3-dihydro-5-phenyl-2H-1,4-benzodiazepin-2-one.

Prepared according to the general procedure (GIII).

### Example XXI

### 1-(2-[4-chloro-2-(fluorescein-5-ylamino)-1,3,5-triazin-6-ylamino]ethyl amino carbonylmethyl)-7-chloro-1,3-dihydro-5-phenyl-2H-1,4-benzodiazepin-2-one

Prepared according to the general procedure (GI).

### Example XXII

### 1-(2-[4-Chloro-2-(fluorescein-6-ylamino)-1,3,5-triazin-6-ylamino]ethyl amino carbonylmethyl)-7-chloro-1,3-dihydro-5-phenyl-2H-1,4-benzodiazepin-2-one (Fig. 16)

Prepared according to the general procedure (GI).

### Example XXIII

### 1-[2-(Fluorescein-5-ylcarbonylamino)ethylamino-carbonylmethyl]-7-chloro-1,3-dihydro-5-phenyl-2H-1,4-benzodiazepin-2-one

Prepared according to the general procedure (GIII).

### Example XXIV

### 1-[2-(Fluorescein-6-ylcarbonylamino)ethylamino-carbonyl-methyl]-7-chloro-1,3-dihydro-5-phenyl-2H-1,4-benzodiazepin-2-one

Prepared according to the general procedure (GII).

### Example XXV

### 1-(3-(2-[Fluorescein-6-ylamino]-4-chloro-1,3,5-triazin-2-ylamino)propyl)-7-chloro-1,3-dihydro-5-phenyl-2H-1,4-benzodiazepin-2-one (Fig. 17)

A solution of 11 micromoles of 1-(3-aminopropyl)-7-chloro-1,3-dihydro-5-phenyl-2H-1, 4-benzodiazepin-2-one and 5.8 mg of 2-(fluorescein-6-ylamino)-4,6-dichloro-1,3,5-triazine in 0.2 ml of methanol was stirred at room temperature for 15 minutes. One equivalent (0.0015 ml) of triethylamine was added,and stirring was continued for 27 hours. The crude product was twice chromatographed on silica gel thin layer plates, once with chloroform-methanol and once with benzene-ethyl acetate-acetone, to give the pure conjugate.

### Example XXVI

### 1-(3-[Fluorescein-6-ylcarbonylamino]propyl)-7-chloro-1,3-dihydro-5-phenyl-2H-1,4-benzodiazepin-2-one (Fig. 18)

A solution of 11 micromoles of 1-(3-aminopropyl)-7-chloro-1,3-dihydro-5-phenyl-2H-1, 4-benzodiazepin-2-one and 5.2 mg of 6-(N-succinimidyloxy-carbonyl) fluorescein in 0.15 ml of dimethylformamide was stirred at room temperature for 4 1/2 hours. Solvent was removed in vacuo and the crude product was purified by chromatographing twice on thin layer silica gel plates, once with chloroform-methanol and once with benzene-ethyl acetate-acetone, to give the pure conjugate.

### Example XXVII

### 1-Methyl-7-[2-(fluorescein-5-ylamino)-4-chloro-1,3,5-triazin-6-ylamino]-1-methyl-7-amino-1,3-dihydro-5-phenyl-2H-1,4-benzodiazepin-2-one (Fig. 20)

The tracer of Figure 20 was prepared in connection with the comparative data of Table I.

A mixture of 13.3 mg of 7-amino-1-methyl-1,3-dihydro-5-phenyl-2H-1,4-benzodiazepin-2-one, 0.021 ml of triethylamine and 26.5 mg of 2-(fluorescein-5-ylamino)-4,6-dichloro-1,3,5-triazine was stirred at ambient temperature overnight in 0.5 ml of methanol. The crude reaction mixture was streaked onto a silica gel thick layer plate and chromatographed with chloroform-methanol to give the pure conjugate.

## Claims (Claims for the following Contracting State(s): AT, BE, CH, DE, FR, GB, GR, IT, LI, LU, NL, SE)

1. A compound comprising the structure: 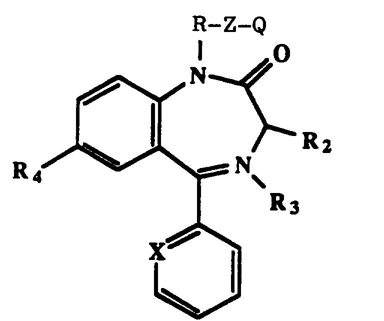
wherein:
X is CH, N or C-halogen;
R₂ is -H or -OH;
R₃ is -O or a nonbonding electron pair;
R₄ is halogen, -NO₂, -NH₂ or -NHCOCH₃;
R is a linking group consisting of from 0 to 20 carbon atoms and heteroatoms, including not more than twelve heteroatoms, arranged in a straight or branched chain and containing up to two ring structures, with the proviso that not more than four heteroatoms may be linked in sequence, nor may more than two sulfur or nitrogen atoms or one oxygen atom be linked in sequence;
Z is C=O, C=NH, NH, NCH₃, -N=N-, SO₂ or CH₂; and
Q is an amino, amido, amidino, urea, thiourea, carbamate, thiocarbamate, triazinylamino, or (carboxyamino)-sulfonamido derivative of fluorescein or Q is 4-chloro-6-fluoresceinylamino-1,3,5-triazin-2-yl or 4-methoxy-6-fluoresceinylamino-1,3,5-triazin-2-yl.

2. The compound of Claim 1 wherein Q is a triazinylamino derivative of fluorescein.

3. The compound of Claim 1 wherein Q is 4-chloro-6-(fluorescein-6-ylamino)-1,3,5-triazin-2-yl, fluorescein-5-yl carbonyl or fluorescein-6-yl carbonyl.

4. The compound of Claim 1 wherein Q is (fluorescein-5-ylamino)carbonyl or (fluorescein-6-ylamino)carbonyl.

5. The compound of Claim 1 wherein Q is (fluorescein-5-yl)amino or (fluorescein-6-yl)amino.

6. A method for making a tracer comprising the step of coupling a precursor of the formula: 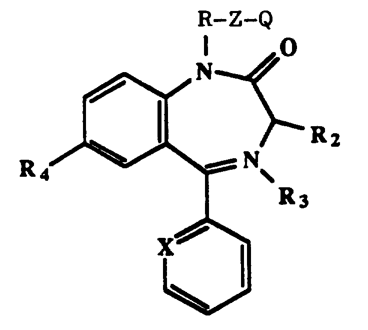
wherein:
X is CH, N or C-halogen;
R₂ is -H or -OH;
R₃ is -O or a nonbonding electron pair;
R₄ is halogen, -NO₂, -NH₂ or -NHCOCH₃;
R is a linking group consisting of from 0 to 20 carbon atoms and heteroatoms, including not more than twelve heteroatoms, arranged in a straight or branched chain and containing up to two ring structures, with the proviso that not more than four heteroatoms may be linked in sequence, nor may more than two sulfur or nitrogen atoms or one oxygen atom be linked in sequence;
Z is NH, CO, SO₂ or -C=NH; and
Q is -H, -OH, or a leaving group,
with fluorescein or a derivative of fluorescein.

7. The method of Claim 6 wherein when ZQ is NH₂, the precursor is coupled by the steps of:
(a) preparing an active ester of carboxyfluorescein;
(b) reacting the active ester with the precursor.

8. The method of Claim 6 wherein when ZQ is CO₂H, the precursor is coupled by the steps of:
(a) preparing an active ester of the precursor;
(b) reacting the active ester with aminofluorescein.

9. The method of Claim 6 wherein ZQ is NH₂ and the precursor is reacted with (4,6-dichloro-1,3,5-triazin-2-ylamino)fluorescein or fluoresceinisothiocyanate.

10. The method of Claim 6 wherein a precursor is coupled by the steps of:
(a) preparing an imidate ester of the precursor; and
(b) reacting the imidate with aminofluorescein.

11. The method of Claim 6 wherein when Q is OH, the precursor is coupled by reacting it with (4,6-dichloro-1,3,5-triazin-2-ylamino)fluorescein.

12. A process for detecting the presence of benzodiazepines and benzodiazepine metabolites in a fluid sample which comprises the steps of:
(a) contacting said sample with antiserum raised against a benzodiazepine linked to an immunogenic carrier substance through its ring nitrogen adjacent to its ring carbonyl and with a compound capable of producing a detectable fluorescence polarization response to the presence of said antiserum, said compound comprising the structure: 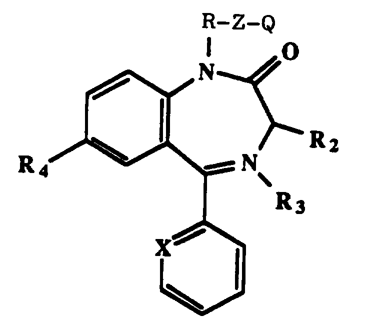
wherein:
X is CH, N or C-halogen;
R₂ is -H or -OH;
R₃ is -O or a nonbonding electron pair;
R₄ is halogen, -NO₂, -NH₂ or -NHCOCH₃;
R is a linking group consisting of from 0 to 20 carbon atoms and heteroatoms, including not more than twelve heteroatoms, arranged in a straight or branched chain and containing up to two ring structures, with the proviso that not more than four heteroatoms may be linked in sequence, nor may more than two sulfur or nitrogen atoms or one oxygen atom be linked in sequence;
Z is C=O, C=NH, NH, NCH₃, -N=N-, SO₂ or CH₂; and
Q is an amino, amido, amidino, urea, thiourea, carbamate, thiocarbamate, triazinylamino, or (carboxyamino)-sulfonamido derivative of fluorescein or Q is 4-chloro-6-fluoresceinylamino-1,3,5-triazin-2-yl or 4-methoxy-6-fluoresceinylamino-1,3,5-triazin-2-yl;
(b) passing plane polarized light through the resulting solution from step (a) to obtain a fluorescence polarization response; and
(c) detecting the fluorescence polarization response of the solution of step (b) as a measure of the presence of benzodiazepines and benzodiazepine metabolites in the sample.

13. The process of Claim 12 wherein the antiserum is raised against an immunogen comprising the structure 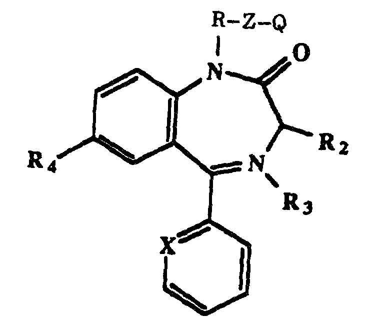
wherein:
X is CH, N or C-halogen;
R₂ is -H or -OH;
R₃ is -O or a nonbonding electron pair;
R₄ is halogen, -NO₂, -NH₂ or -NHCOCH₃;
R is a linking group consisting of from 0 to 20 carbon atoms and heteroatoms, including not more than twelve heteroatoms, arranged in a straight or branched chain and containing up to two ring structures, with the proviso that not more than four heteroatoms may be linked in sequence, nor may more than two sulfur or nitrogen atoms or one oxygen atom be linked in sequence;
Z is C=O, C=NH, NH, NCH₃, -N=N-, SO₂ or CH₂; and
Q is an immunogenic carrier substance.

14. The process of Claim 13 wherein in the formula of said immunogen R₄ is Cl and Z is C=O, C=NH, SO₂, NH, NCH₃ or CH₂.

15. The process of Claim 12 wherein the antiserum is raised against the conjugate of 1-carboxymethyl-7-chloro-1,3-dihydro-5-phenyl-2H-1,4-benzodiazepine-2-one and bovine serum albumin.

16. The process of Claim 15 wherein the antiserum was raised in sheep and the sample is human urine.

17. The process of Claim 15 wherein the compound of Claim 1 is a mixture of two compounds in which R₂ is hydrogen, R₃ is not O, R₄ is Cl, X is CH, Z is NH, Q is 4-chloro-6-(fluorescein-6-ylamino)-1,3,5-triazin-2-yl, and R is either -CH₂CH₂- or -CH₂CONHCH₂CH₂-.

18. An antibody raised against the conjugate of 1-carboxymethyl-7-chloro-1,3-dihydro-5-phenyl-2H-1,4-benzodiazepine-2-one and bovine serum albumin.

19. The antibody of Claim 18 which was raised in sheep or rabbit.

20. The compound of Claim 1 wherein R₂ is hydrogen, R₃ is not O, R₄ is Cl, X is CH, Z is NH, Q is 4-chloro-6-(fluorescein-6-ylamino)-1,3,5-triazin-2-yl and R is either -CH₂CH₂- or -CH₂CONHCH₂CH₂-.

21. Process for preparing an antibody of Claim 18 comprising the steps of administering the conjugate of 1-carboxymethyl-7-chloro-1,3-dihydro-5-phenyl-2H-1,4-benzodiazepine-2-one and bovine serum albumin to nonhuman animals or to in vitro cultures of immunocompetent cells by a series of inoculations, said inoculations eliciting the production of said antibodies.

## Claims (Claims for the following Contracting State(s): ES)

1. A method for making a tracer comprising the step of coupling a precursor of the formula: 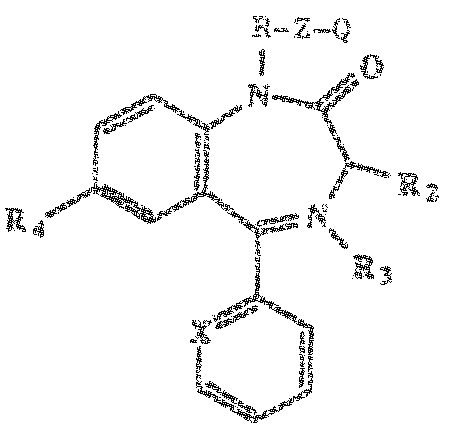
wherein:
X is CH, N or C-halogen;
R₂ is -H or -OH;
R₃ is -O or a nonbonding electron pair;
R₄ is halogen, -NO₂, -NH₂ or -NHCOCH₃;
R is a linking group consisting of from 0 to 20 carbon atoms and heteroatoms, including not more than twelve heteroatoms, arranged in a straight or branched chain and containing up to two ring structures, with the proviso that not more than four heteroatoms may linked in sequence, nor may more than two sulfur or nitrogen atoms or one oxygen atom be linked in sequence;
Z is NH, CO, SO₂ or -C=NH; and
Q is -H, -OH, or a leaving group,
with fluorescein or a derivative of fluorescein.

2. The method of Claim 1 wherein when ZQ is NH₂, the precursor is coupled by the steps of:
(a) preparing an active ester of carboxyfluorescein;
(b) reacting the active ester with the precursor.

3. The method of Claim 1 wherein when ZQ is CO₂H, the precursor is coupled by the steps of:
(a) preparing an active ester of the precursor;
(b) reacting the active ester with aminofluorescein.

4. The method of Claim 1 wherein ZQ is NH₂ and the precursor is reacted with (4,6-dichloro-1,3,5-triazin-2-ylamino)fluorescein or fluoresceinisothiocyanate.

5. The method of Claim 1 wherein a precursor is coupled by the steps of:
(a) preparing an imidate ester of the precursor; and
(b) reacting the imidate with aminofluorescein.

6. The method of Claim 1 wherein when Q is OH, the precursor is coupled by reacting it with (4,6-dichloro-1,3,5-triazin-2-ylamino)fluorescein.

7. A process for detecting the presence of benzodiazepines and benzodiazepine metabolites in a fluid sample which comprises the steps of:
(a) contacting said sample with antiserum raised against a benzodiazepine linked to an immunogenic carrier substance through its ring nitrogen adjacent to its ring carbonyl and with a compound capable of producing a detectable fluorescence polarization response to the presence of said antiserum, said compound comprising the structure: 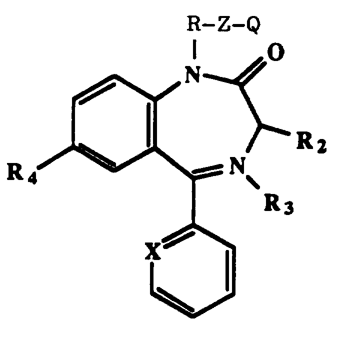
wherein:
X is CH, N or C-halogen;
R₂ is -H or -OH;
R₃ is -O or a nonbonding electron pair;
R₄ is halogen, -NO₂, -NH₂ or -NHCOCH₃;
R is a linking group consisting of from 0 to 20 carbon atoms and heteroatoms, including not more than twelve heteroatoms, arranged in a straight or branched chain and containing up to two ring structures, with the proviso that not more than four heteroatoms may be linked in sequence, nor may more than two sulfur or nitrogen atoms or one oxygen atom be linked in sequence;
Z is C=O, C=NH, NH, NCH₃, -N=N-, SO₂ or CH₂; and
Q is an amino, amido, amidino, urea, thiourea, carbamate, thiocarbamate, triazinylamino, or (carboxyamino)-sulfonamido derivative of fluorescein or Q is 4-chloro-6-fluoresceinylamino-1,3,5-triazin-2-yl or 4-methoxy-6-fluoresceinylamino-1,3,5-triazin-2-yl;
(b) passing plane polarized light through the resulting solution from step (a) to obtain a fluorescence polarization response; and
(c) detecting the fluorescence polarization response of the solution of step (b) as a measure of the presence of benzodiazepines and benzodiazepine metabolites in the sample.

8. The process of Claim 7 wherein Q is a triazinylamino derivative of fluorescein.

9. The process of Claim 7 wherein Q is 4-chloro-6-(fluorescein-6-ylamino)-1,3,5-triazin-2-yl, fluorescein-5-yl carbonyl or fluorescein-6-yl carbonyl.

10. The process of Claim 7 wherein Q is (fluorescein-5-ylamino)carbonyl or (fluorescein-6-ylamino)carbonyl.

11. The process of Claim 7 wherein Q is (fluorescein-5-yl)amino or (fluorescein-6-yl)amino.

12. The process of Claim 7 wherein the antiserum is raised against an immunogen comprising the structure 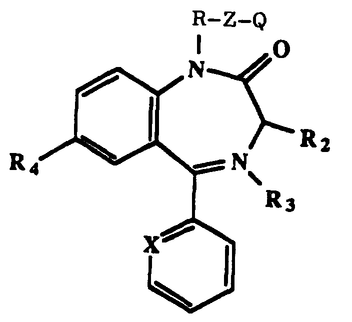
wherein:
X is CH, N or C-halogen;
R₂ is -H or -OH;
R₃ is -O or a nonbonding electron pair;
R₄ is halogen, -NO₂, -NH₂ or -NHCOCH₃;
R is a linking group consisting of from 0 to 20 carbon atoms and heteroatoms, including not more than twelve heteroatoms, arranged in a straight or branched chain and containing up to two ring structures, with the proviso that not more than four heteroatoms may be linked in sequence, nor may more than two sulfur or nitrogen atoms or one oxygen atom be linked in sequence;
Z is C=O, C=NH, NH, NCH₃, -N=N-, SO₂ or CH₂; and
Q is an immunogenic carrier substance.

13. The process of Claim 12 wherein in the formula of said immunogen R₄ is Cl and Z is C=O, C=NH, SO₂, NH, NCH₃ or CH₂.

14. The process of Claim 12 wherein the antiserum is raised against the conjugate of 1-carboxymethyl-7-chloro-1,3-dihydro-5-phenyl-2H-1,4-benzodiazepine-2-one and bovine serum albumin.

15. The process of Claim 14 wherein the antiserum is raised in sheep or rabbit.

16. The process of Claim 14 wherein the antiserum was raised in sheep and the sample is human urine.

17. The process of Claim 14 wherein the compound of Claim 12 is a mixture of two compounds in which R₂ is hydrogen, R₃ is not O, R₄ is Cl, X is CH, Z is NH, Q is 4-chloro-6-(fluorescein-6-ylamino)-1,3,5-triazin-2-yl, and R is either -CH₂CH₂- or -CH₂CONHCH₂CH₂-.

18. Process for preparing an antibody comprising the steps of administering the conjugate of 1-carboxymethyl-7-chloro-1,3-dihydro-5-phenyl-2H-1,4-benzodiazepine-2-one and bovine serum albumin to nonhuman animals or to in vitro cultures of immunocompetent cells by a series of inoculations, said inoculations eliciting the production of said antibodies.

19. The process of Claim 18 wherein the conjugate was administered to sheep or rabbits.

## Patentansprüche (Patentansprüche für folgende(n) Vertragsstaat(en): AT, BE, CH, DE, FR, GB, GR, IT, LI, LU, NL, SE)

1. Verbindung, die folgende Struktur umfaßt: 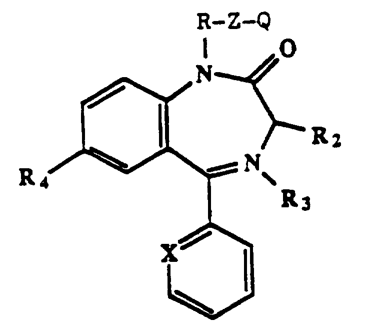
wobei:
X gleich CH, N oder C-Halogen ist;
R₂ gleich -H oder -OH ist;
R₃ gleich -O oder ein nichtbindendes Elektronenpaar ist;
R₄ gleich Halogen, -NO₂, -NH₂ oder -NHCOCH₃ ist;
R eine verbindende Gruppe aus 0 bis 20 Kohlenstoffatomen und Heteroatomen mit nicht mehr als zwölf Heteroatomen ist, die in durchgehender oder verzweigter Kette angeordnet sind und bis zu zwei Ringstrukturen enthalten, unter dem Vorbehalt, daß weder mehr als vier Heteroatome in Folge verknüpft sind, noch mehr als zwei Schwefel- oder Stickstoffatome oder ein Sauerstoffatom in Folge verknüpft sind;
Z gleich C=O, C=NH, NH, NCH₃, -N=N-, SO₂ oder CH₂ ist, und
Q ein Amino-, Amido-, Amidino-, Harnstoff-, Thioharnstoff-, Carbamat-, Thiocarbamat-, Triazinylamino- oder (Carboxyamino)-sulfonamido-Derivat von Fluorescein ist, oder Q gleich 4-Chlor-6-fluoresceinylamino-1,3,5-triazin-2-yl oder 4-Methoxy-6-fluoresceinylamino-1,3,5-triazin-2-yl ist.

2. Verbindung nach Anspruch 1, wobei Q ein Triazinylaminoderivat von Fluorescein ist.

3. Verbindung nach Anspruch 1, wobei Q gleich 4-Chlor-6-(fluorescein-6-ylamino)-1,3,5-triazin-2-yl Fluorescein-5-ylcarbonyl oder Fluorescein-6-ylcarbonyl ist.

4. Verbindung nach Anspruch 1, wobei Q gleich (Fluorescein-5-ylamino)carbonyl oder (Fluorescein-6-ylamino)carbonyl ist.

5. Verbindung nach Anspruch 1, wobei Q gleich (Fluorescein-5-yl)amino oder (Fluorescein-6-yl)amino ist.

6. Verfahren zur Herstellung eines Tracers, das den Schritt der Kupplung eines Vorläufers nach folgender Formel, 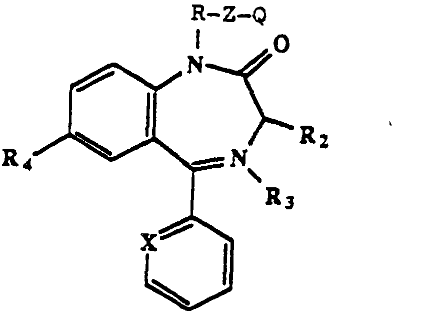
wobei:
X gleich CH, N oder C-Halogen ist;
R₂ gleich -H oder -OH ist;
R₃ gleich -O oder ein nichtbindendes Elektronenpaar ist;
R₄ gleich Halogen, -NO₂, -NH₂ oder -NHCOCH₃ ist;
R eine verbindende Gruppe aus 0 bis 20 Kohlenstoffatomen und Heteroatomen mit nicht mehr als zwölf Heteroatomen ist, die in durchgehender oder verzweigter Kette angeordnet sind und bis zu zwei Ringstrukturen enthalten, unter dem Vorbehalt, daß weder mehr als vier Heteroatome in Folge verknüpft sind, noch mehr als zwei Schwefel- oder Stickstoffatome oder ein Sauerstoffatom in Folge verknüpft sind;
Z gleich NH, CO, SO₂ oder -C=NH ist, und
Q gleich -H, -OH oder eine Austrittsgruppe ist, mit Fluorescein oder einem Derivat von Fluorescein umfaßt.

7. Verfahren nach Anspruch 6, wobei ZQ gleich NH₂ ist, wobei die Kupplung des Vorläufers in folgenden Schritten durchgeführt wird:
(a) Herstellung eines aktiven Esters von Carboxyfluorescein;
(b) Umsetzung des aktiven Esters mit dem Vorläufer.

8. Verfahren nach Anspruch 6, wobei ZQ gleich CO₂H ist, wobei die Kupplung des Vorläufers in folgenden Schritten durchgeführt wird:
(a) Herstellung eines aktiven Esters des Vorläufers;
(b) Umsetzung des aktiven Esters mit Aminofluorescein.

9. Verfahren nach Anspruch 6, wobei ZQ gleich NH₂ ist und der Vorläufer mit (4,6-Dichlor-1,3,5-triazin-2-ylamino)fluorescein oder Fluoresceinisothiocyanat umgesetzt wird.

10. Verfahren nach Anspruch 6, wobei die Kupplung des Vorläufers durch folgende Schritte durchgeführt wird:
(a) Herstellung eines Imidatesters des Vorläufers;
(b) Umsetzung des Imidats mit Aminofluorescein.

11. Verfahren nach Anspruch 6, wobei, falls Q gleich OH ist, der Vorläufer durch Umsetzung mit (4,6-Dichlor-1,3,5-triazin-2-ylamino)fluorescein gekuppelt wird.

12. Verfahren zum Nachweis der Anwesenheit von Benzodiazepinen und Benzodiazepin-Metaboliten in einer flüssigen Probe, das folgende Schritte umfaßt:
(a) in-Kontakt-Bringen dieser Probe mit Antiserum, das gegen ein Benzodiazepin gezüchtet wurde, das über seinen Ringstickstoff neben seinem Ringcarbonylatom an eine immunogene Trägersubstanz gebunden ist, und mit einer Verbindung, die in der Lage ist, eine nachweisbare Fluoreszenzpolarisationsantwort auf die Anwesenheit dieses Antiserums hervorzurufen, wobei diese Verbindung folgende Struktur umfaßt, 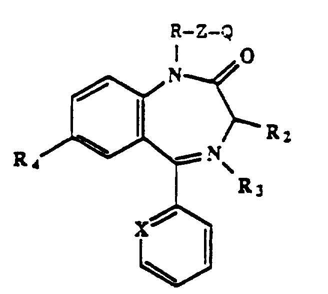
wobei:
X gleich CH, N oder C-Halogen ist;
R₂ gleich -H oder -OH ist;
R₃ gleich -O oder ein nichtbindendes Elektronenpaar ist;
R₄ gleich Halogen, -NO₂, -NH₂ oder -NHCOCH₃ ist;
R eine verbindende Gruppe aus 0 bis 20 Kohlenstoffatomen und Heteroatomen mit nicht mehr als zwölf Heteroatomen ist, die in durchgehender oder verzweigter Kette angeordnet sind und bis zu zwei Ringstrukturen enthalten, unter dem Vorbehalt, daß weder mehr als vier Heteroatome in Folge verknüpft sind, noch mehr als zwei Schwefel- oder Stickstoffatome oder ein Sauerstoffatom in Folge verknüpft sind;
Z gleich C=O, C=NH, NH, NCH₃, -N=N-, SO₂ oder CH₂ ist, und
Q ein Amino-, Amido-, Amidino-, Harnstoff-, Thioharnstoff-, Carbamat-, Thiocarbamat-, Triazinylamino- oder (Carboxyamino)-sulfonamido-Derivat von Fluorescein ist, oder Q gleich 4-chlor-6-fluoresceinylamino-1,3,5-triazin-2-yl oder 4-Methoxy-6-fluoresceinylamino-1,3,5-triazin-2-yl ist;
(b) Hindurchtretenlassen planar polarisierten Lichtes durch die resultierende Lösung nach Schritt (a), um eine Fluoreszenzpolarisationsantwort zu erhalten; und
(c) Detektion der Fluoreszenzpolarisationsantwort der Lösung nach Schritt (b) als Maß für die Anwesenheit von Benzodiazepinen oder Benzodiazepin-Metaboliten in der Probe.

13. Verfahren nach Anspruch 12, wobei das Antiserum gegen ein Immunogen mit der folgenden Struktur gezüchtet wird: 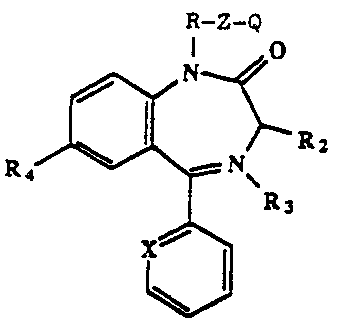
wobei:
X gleich CH, N oder C-Halogen ist;
R₂ gleich -H oder -OH ist;
R₃ gleich -O oder ein nichtbindendes Elektronenpaar ist;
R₄ gleich Halogen, -NO₂, -NH₂ oder -NHCOCH₃ ist;
R eine verbindende Gruppe aus 0 bis 20 Kohlenstoffatomen und Heteroatomen mit nicht mehr als zwölf Heteroatomen ist, die in durchgehender oder verzweigter Kette angeordnet sind und bis zu zwei Ringstrukturen enthalten, unter dem Vorbehalt, daß weder mehr als vier Heteroatome in Folge verknüpft sind, noch mehr als zwei Schwefel- oder Stickstoffatome oder ein Sauerstoffatom in Folge verknüpft sind;
Z gleich C=O, C=NH, NH, NCH₃, -N=N-, SO₂ oder CH₂ ist, und
Q eine immunogene Trägersubstanz ist.

14. Verfahren nach Anspruch 13, wobei in der Formel des Immunogens R₄ gleich Cl und Z gleich C=O, C=NH, SO₂, NH, NCH₃ oder CH₂ ist.

15. Verfahren nach Anspruch 13, wobei das Antiserum gegen das Konjugat von 1-carboxymethyl-7-chlor-1,3-dihydro-5-phenyl-2H-1,4-benzodiazepin-2-on und Rinderserumalbumin gezüchtet wird.

16. Verfahren nach Anspruch 15, wobei das Antiserum in Schafen gezüchtet wird, und die Probe Humanurin ist.

17. Verfahren nach Anspruch 15, wobei die Verbindung nach Anspruch 1 ein Gemisch aus zwei Verbindungen ist, wobei R₂ gleich Wasserstoff ist, R₃ ungleich O ist, R₄ gleich Cl ist, X gleich CH ist, Z gleich NH ist, Q gleich 4-Chlor-6-(fluorescein-6-ylamino)-1,3,5-triazin-2-yl ist, und R entweder gleich -CH₂CH₂-oder gleich -CH₂CONHCH₂CH₂- ist.

18. Antikörper, der gegen das Konjugat aus 1-carboxymethyl-7-chlor-1,3-dihydro-5-phenyl-2H-1,4-benzodiazepin-2-on und Rinderserumalbumin gezüchtet wird.

19. Antikörper nach Anspruch 18, der in Schafen oder Kaninchen gezüchtet wird.

20. Verbindung nach Anspruch 1, wobei R₂ gleich Wasserstoff ist, R₃ ungleich O ist, R₄ gleich Cl ist, X gleich CH ist, Z gleich NH ist, Q gleich 4-Chlor-6-(fluorescein-6-ylamino)-1,3,5-triazin-2-yl ist, und R entweder gleich -CH₂CH₂- oder gleich -CH₂CONHCH₂CH₂-ist.

21. Verfahren zur Herstellung eines Antikörpers nach Anspruch 18, umfassend den Schritt der Verabreichung des Konjugats aus 1-Carboxymethyl-7-chlor-1,3-dihydro-5-phenyl-2H-1,4-benzodiazepin-2-on und Rinderserumalbumin an Tiere oder an in vitro -Kulturen von immunkompetenten Zellen mittels einer Reihe von Animpfungen, wobei diese Animpfungen die Bildung dieses Antikörpers auslösen.

## Patentansprüche (Patentansprüche für folgende(n) Vertragsstaat(en): ES)

1. Verfahren zur Herstellung eines Tracers, das den Schritt der Kupplung eines Vorläufers nach folgender Formel, 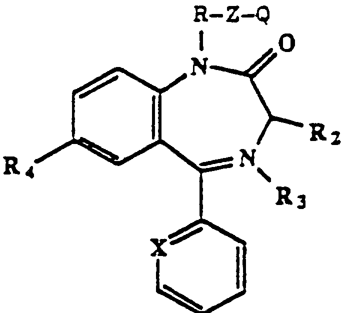
wobei:
X gleich CH, N oder C-Halogen ist;
R₂ gleich -H oder -OH ist;
R₃ gleich -O oder ein nichtbindendes Elektronenpaar ist;
R₄ gleich Halogen, -NO₂, -NH₂ oder -NHCOCH₃ ist;
R eine verbindende Gruppe aus 0 bis 20 Kohlenstoffatomen und Heteroatomen mit nicht mehr als zwölf Heteroatomen ist, die in durchgehender oder verzweigter Kette angeordnet sind und bis zu zwei Ringstrukturen enthalten, unter dem Vorbehalt, daß weder mehr als vier Heteroatome in Folge verknüpft sind, noch mehr als zwei Schwefel- oder Stickstoffatome oder ein Sauerstoffatom in Folge verknüpft sind;
Z gleich NH, CO, SO₂ oder -C=NH ist, und
Q gleich -H, -OH oder eine Austrittsgruppe ist, mit Fluorescein oder einem Derivat von Fluorescein umfaßt.

2. Verfahren nach Anspruch 1, wobei ZQ gleich NH₂ ist, wobei die Kupplung des Vorläufers in folgenden Schritten durchgeführt wird:
(a) Herstellung eines aktiven Esters von Carboxyfluorescein;
(b) Umsetzung des aktiven Esters mit dem Vorläufer.

3. Verfahren nach Anspruch 1, wobei ZQ gleich CO₂H ist, wobei die Kupplung des Vorläufers in folgenden Schritten durchgeführt wird:
(a) Herstellung eines aktiven Esters des Vorläufers;
(b) Umsetzung des aktiven Esters mit Aminofluorescein.

4. Verfahren nach Anspruch 1, wobei ZQ gleich NH₂ ist und der Vorläufer mit (4,6-Dichlor-1,3,5-triazin-2-ylamino) fluorescein oder Fluoresceinisothiocyanat umgesetzt wird.

5. Verfahren nach Anspruch 1, wobei die Kupplung des Vorläufers durch folgende Schritte durchgeführt wird:
(a) Herstellung eines Imidatesters des Vorläufers;
(b) Umsetzung des Imidats mit Aminofluorescein.

6. Verfahren nach Anspruch 1, wobei, falls Q gleich OH ist, der Vorläufer durch Umsetzung mit (4,6-Dichlor-1,3,5-triazin-2-ylamino)fluorescein gekuppelt wird.

7. Verfahren zum Nachweis der Anwesenheit von Benzodiazepinen und Benzodiazepin-Metaboliten in einer flüssigen Probe, das folgende Schritte umfaßt:
(a) in-Kontakt-Bringen dieser Probe mit Antiserum, das gegen ein Benzodiazepin gezüchtet wurde, das über seinen Ringstickstoff neben seinem Ringcarbonylatom an eine immunogene Trägersubstanz gebunden ist, und mit einer Verbindung, die in der Lage ist, eine nachweisbare Fluoreszenzpolarisationsantwort auf die Anwesenheit dieses Antiserums hervorzurufen, wobei diese Verbindung folgende Struktur umfaßt, 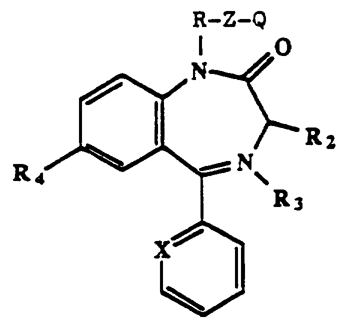
wobei:
X gleich CH, N oder C-Halogen ist;
R₂ gleich -H oder -OH ist;
R₃ gleich -O oder ein nichtbindendes Elektronenpaar ist;
R₄ gleich Halogen, -NO₂, -NH₂ oder -NHCOCH₃ ist;
R eine verbindende Gruppe aus 0 bis 20 Kohlenstoffatomen und Heteroatomen mit nicht mehr als zwölf Heteroatomen ist, die in durchgehender oder verzweigter Kette angeordnet sind und bis zu zwei Ringstrukturen enthalten, unter dem Vorbehalt, daß weder mehr als vier Heteroatome in Folge verknüpft sind, noch mehr als zwei Schwefel- oder Stickstoffatome oder ein Sauerstoffatom in Folge verknüpft sind;
Z gleich C=O, C=NH, NH, NCH₃, -N=N-, SO₂ oder CH₂ ist, und
Q ein Amino-, Amido-, Amidino-, Harnstoff-, Thioharnstoff-, Carbamat-, Thiocarbamat-, Triazinylamino- oder (Carboxyamino)-sulfonamido-Derivat von Fluorescein ist, oder Q gleich 4-Chlor-6-fluoresceinylamino-1,3,5-triazin-2-yl oder 4-Methoxy-6-fluoresceinylamino-1,3,5-triazin-2-yl ist;
(b) Hindurchtretenlassen planar polarisierten Lichtes durch die resultierende Lösung nach Schritt (a), um eine Fluoreszenzpolarisationsantwort zu erhalten; und
(c) Detektion der Fluoreszenzpolarisationsantwort der Lösung nach Schritt (b) als Maß für die Anwesenheit von Benzodiazepinen oder Benzodiazepin-Metaboliten in der Probe.

8. Verfahren nach Anspruch 7, wobei Q ein Triazinylaminoderivat von Fluorescein ist.

9. Verfahren nach Anspruch 7, wobei Q gleich 4-Chlor-6-(fluorescein-6-ylaminol-1,3,5-triazin-2-yl, Fluorescein-5-ylcarbonyl oder Fluorescein-6-ylcarbonyl ist.

10. Verfahren nach Anspruch 7, wobei Q gleich (Fluorescein-5-ylamino)carbonyl oder (Fluorescein-6-ylamino)carbonyl ist.

11. Verfahren nach Anspruch 7, wobei Q gleich (Fluorescein-5-yl)amino oder (Fluorescein-6-yl)amino ist.

12. Verfahren nach Anspruch 7, wobei das Antiserum gegen ein Immunogen mit der folgenden Struktur gezüchtet wird: 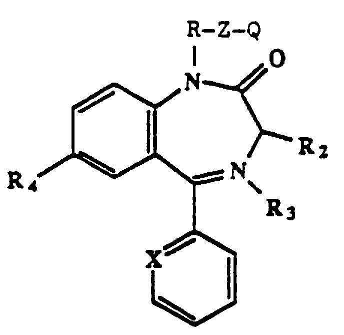
wobei:
X gleich CH, N oder C-Halogen ist;
R₂ gleich -H oder -OH ist;
R₃ gleich -O oder ein nichtbindendes Elektronenpaar ist;
R₄ gleich Halogen, -NO₂, -NH₂ oder -NHCOCH₃ ist;
R eine verbindende Gruppe aus 0 bis 20 Kohlenstoffatomen und Heteroatomen mit nicht mehr als zwölf Heteroatomen ist, die in durchgehender oder verzweigter Kette angeordnet sind und bis zu zwei Ringstrukturen enthalten, unter dem Vorbehalt, daß weder mehr als vier Heteroatome in Folge verknüpft sind, noch mehr als zwei Schwefel- oder Stickstoffatome oder ein Sauerstoffatom in Folge verknüpft sind;
Z gleich C=O, C=NH, NH, NCH₃, -N=N-, SO₂ oder CH₂ ist, und
Q eine immunogene Trägersubstanz ist.

13. Verfahren nach Anspruch 12, wobei in der Formel des Immunogens R₄ gleich Cl und Z gleich C=O, C=NH, SO₂, NH, NCH₃ oder CH₂ ist.

14. Verfahren nach Anspruch 12, wobei das Antiserum gegen das Konjugat von 1-Carboxymethyl-7-chlor-1,3-dihydro-5-phenyl-2H-1,4-benzodiazepin-2-on und Rinderserumalbumin gezüchtet wird.

15. Verfahren nach Anspruch 14, wobei das Antiserum in Schafen oder Kaninchen gezüchtet wird.

16. Verfahren nach Anspruch 14, wobei das Antiserum in Schafen gezüchtet wird, und die Probe Humanurin ist.

17. Verfahren nach Anspruch 14, wobei die Verbindung nach Anspruch 1 ein Gemisch aus zwei Verbindungen ist, wobei R₂ gleich Wasserstoff ist, R₃ ungleich O ist, R₄ gleich Cl ist, X gleich CH ist, Z gleich NH ist, Q gleich 4-Chlor-6-(fluorescein-6-ylamino)-1,3,5-triazin-2-yl ist, und R entweder gleich -CH₂CH₂-oder gleich -CH₂CONHCH₂CH₂- ist.

18. Verfahren zur Herstellung eines Antikörpers, umfassend den Schritt der Verabreichung des Konjugats aus 1-Carboxymethyl-7-chlor-1,3-dihydro-5-phenyl-2H-1,4-benzodiazepin-2-on und Rinderserumalbumin an Tiere oder an in vitro -Kulturen von immunkompetenten Zellen mittels einer Reihe von Animpfungen, wobei diese Animpfungen die Bildung dieses Antikörpers auslösen.

19. Verfahren nach Anspruch 18, wobei das Konjugat Schafen oder Kaninchen verabreicht wird.

## Revendications (Revendications pour l'(les) Etat(s) contractant(s) suivant(s): AT, BE, CH, DE, FR, GB, GR, IT, LI, LU, NL, SE)

1. Composé comprenant la structure : 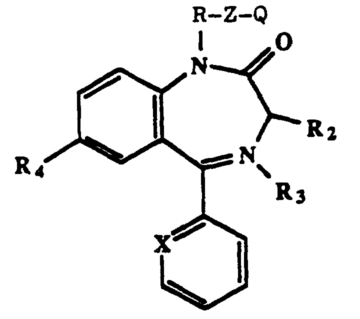
dans laquelle :
X est CH, N ou C-halogène,
R₂ est -H ou -OH,
R₃ est -O ou une paire d'électrons non liants,
R₄ est halogène, -NO₂, -NH₂ ou -NHCOCH₃,
R est un groupe de liaison consistant en 0 à 20 atomes de carbone et hétéroatomes, ne comprenant pas plus de 12 hétéroatomes, disposé en une chaîne linéaire ou ramifiée et contenant jusqu'à deux structures cycliques, à condition que pas plus de quatre hétéroatomes puissent être liés successivement et que pas plus de deux atomes de soufre ou d'azote ou un atome d'oxygène puissent être liés successivement,
Z est C=O, C=NH, NH, NCH₃, -N=N-, SO₂ ou CH₂, et
Q est un dérivé amino, amido, amidino, urée, thiourée, carbamate, thiocarbamate, triazinylamino ou (carboxyamino)-sulfonamido de fluorescéine ou Q est 4-chloro-6-fluorescéinylamino-1,3,5-triazin-2-yle ou 4-méthoxy-6-fluorescéinylamino-1,3,5-triazin-2-yle.

2. Composé selon la revendication 1 dans lequel Q est un dérivé triazinylamino de fluorescéine.

3. Composé selon la revendication dans lequel Q est 4-chloro-6-(fluorescéin-6-ylamino)-1,3,5-triazin-2-yle, fluorescéin-5-ylcarbonyle ou fluorescéin-6-ylcarbonyle.

4. Composé selon la revendication 1 dans lequel Q est (fluorescéin-5-ylamino)carbonyle ou (fluorescéin-6-ylamino)carbonyle.

5. Composé selon la revendication 1 dans lequel Q est (fluorescéin-5-yl)amino ou (fluorescéin-6-yl)amino.

6. Procédé pour préparer un traceur comprenant l'étape de couplage d'un précurseur de formule : 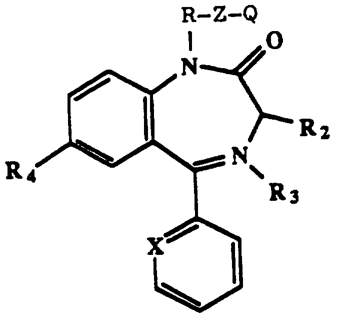
dans laquelle :
X est CH, N ou C-halogène,
R₂ est -H ou -OH,
R₃ est -O ou une paire d'électrons non liants,
R₄ est halogène, -NO₂, -NH₂ ou -NHCOCH₃,
R est un groupe de liaison consistant en 0 à 20 atomes de carbone et hétéroatomes, ne comprenant pas plus de 12 hétéroatomes, disposé en une chaîne linéaire ou ramifiée et contenant jusqu'à deux structures cycliques, à condition que pas plus de quatre hétéroatomes puissent être liés successivement et que pas plus de deux atomes de soufre ou d'azote ou un atome d'oxygène puissent être liés successivement,
Z est NH, CO, SO₂ ou -C=NH, et
Q est -H, -OH ou un groupe partant,
avec la fluorescéine ou un dérivé de la fluorescéine.

7. Procédé selon la revendication 6 dans lequel, lorsque ZQ est NH₂, le précurseur est couplé par les étapes de :
(a) préparation d'un ester actif de carboxyfluorescéine,
(b) réaction de l'ester actif avec le précurseur.

8. Procédé selon la revendication 6 dans lequel, lorsque ZQ est CO₂H, le précurseur est couplé par les étapes de :
(a) préparation d'un ester actif du précurseur,
(b) réaction de l'ester actif avec l'aminofluorescéine.

9. Procédé selon la revendication 6 dans lequel ZQ est NH₂ et le précurseur est mis à réagir avec la (4,6-dichloro-1,3,5-triazin-2-ylamino)-fluorescéine ou l'isothiocyanate de fluorescéine.

10. Procédé selon la revendication 6 dans lequel un précurseur est couplé par les étapes de :
(a) préparation d'un ester imidate du précurseur,
(b) réaction de l'imidate avec l'aminofluorescéine.

11. Procédé selon la revendication 6 dans lequel, lorsque Q est OH, le précurseur est couplé par réaction du précurseur avec la (4,6-dichloro-1,3,5-triazin-2-ylamino)fluorescéine.

12. Procédé pour détecter la présence de benzodiazépines et de métabolites de benzodiazépines dans un échantillon de liquide qui comprend les étapes de :
(a) mise en contact dudit échantillon avec un antisérum produit contre une benzodiazépine liée à une substance support immunogène par son azote cyclique adjacent à son carbonyle cyclique et avec un composé capable de produire une réponse de polarisation de fluorescence détectable à la présence dudit antisérum, ledit composé comprenant la structure : 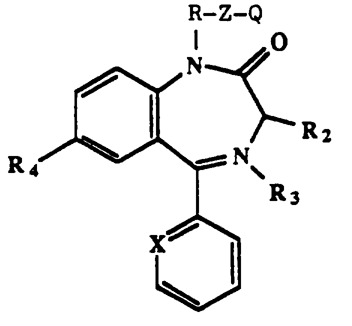
dans laquelle :
X est CH, N ou C-halogène,
R₂ est -H ou -OH,
R₃ est -O ou une paire d'électrons non liants,
R₄ est halogène, -NO₂, -NH₂ ou -NHCOCH₃,
R est un groupe de liaison consistant en 0 à 20 atomes de carbone et hétéroatomes, ne comprenant pas plus de 12 hétéroatomes, disposé en une chaîne linéaire ou ramifiée et contenant jusqu'à deux structures cycliques, à condition que pas plus de quatre hétéroatomes puissent être liés successivement et que pas plus de deux atomes de soufre ou d'azote ou un atome d'oxygène puissent être liés successivement,
Z est C=O, C=NH, NH, NCH₃, -N=N-, SO₂ ou CH₂, et
Q est un dérivé amino, amide, amidino, urée, thiourée, carbamate, thiocarbamate, triazinylamino ou (carboxyamino)-sulfonamido de fluorescéine ou Q est 4-chloro-6-fluorescéinylamino-1,3,5-triazin-2-yle ou 4-méthoxy-6-fluorescéinylamino-1,3,5-triazin-2-yle,
(b) passage d'une lumière polarisée dans un plan dans la solution résultante provenant de l'étape (a) pour obtenir une réponse de polarisation de fluorescence, et
(c) détection de la réponse de polarisation de fluorescence de la solution de l'étape (b) en tant que mesure de la présence de benzodiazépines et de métabolites de benzodiazépines dans l'échantillon.

13. Procédé selon la revendication 12 dans lequel l'antisérum est produit contre un immunogène comprenant la structure : 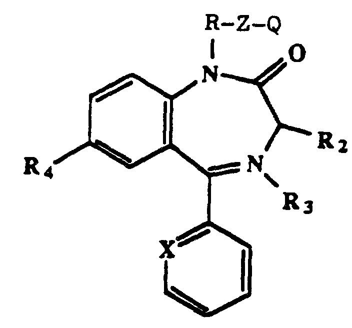
dans laquelle :
X est CH, N ou C-halogène,
R₂ est -H ou -OH,
R₃ est -O ou une paire d'électrons non liants,
R₄ est halogène, -NO₂, -NH₂ ou -NHCOCH₃,
R est un groupe de liaison consistant en 0 à 20 atomes de carbone et hétéroatomes, ne comprenant pas plus de 12 hétéroatomes, disposé en une chaîne linéaire ou ramifiée et contenant jusqu'à deux structures cycliques, à condition que pas plus de quatre hétéroatomes puissent être liés successivement et que pas plus de deux atomes de soufre ou d'azote ou un atome d'oxygène puissent être liés successivement,
Z est C=O, C=NH, NH, NCH₃, -N=N-, SO₂ ou CH₂, et
Q est une substance support immunogène.

14. Procédé selon la revendication 13 dans lequel, dans la formule dudit immunogène, R₄ est Cl et Z est C=O, C=NH, SO₂, NH, NCH₃ ou CH₂.

15. Procédé selon la revendication 12 dans lequel l'antisérum est produit contre le conjugué de 1-carboxyméthyl-7-chloro-1,3-dihydro-5-phényl-2H-1,4-benzodiazépin-2-one et de sérumalbumine bovine.

16. Procédé selon la revendication 15 dans lequel l'antisérum a été produit chez le mouton et l'échantillon est de l'urine humaine.

17. Procédé selon la revendication 15 dans lequel le composé selon la revendication est un mélange de deux composés dans lesquels R₂ est l'hydrogène, R₃ n'est pas O, R₄ est Cl, X est CH, Z est NH, Q est 4-chloro-6-(fluorescéin-6-ylamino)-1,3,5-triazin-2-yle et R est -CH₂CH₂- ou -CH₂CONHCH₂CH₂-.

18. Anticorps produit contre le conjugué de 1-carboxyméthyl-7-chloro-1,3-dihydro-5-phényl-2H-1,4-benzodiazépin-2-one et de sérumalbumine bovine.

19. Anticorps selon la revendication 18 qui a été produit chez le mouton ou le lapin.

20. Composé selon la revendication 1 dans lequel R₂ est l'hydrogène, R₃ n'est pas O, R₄ est Cl, X est CH, Z est NH, Q est 4-chloro-6-(fluorescéin-6-ylamino)-1,3,5-triazin-2-yle et R est -CH₂CH₂- ou -CH₂CONHCH₂CH₂-.

21. Procédé de préparation d'un anticorps selon la revendication 18 comprenant les étapes d'administration du conjugué de 1-carboxyméthyl-7-chloro-1,3-dihydro-5-phényl-2H-1,4-benzodiazépin-2-one et de sérumalbumine bovine à des animaux non humains ou à des cultures in vitro de cellules immunocompétentes par une série d'inoculations, lesdites inoculations déclenchant la production desdits anticorps.

## Revendications (Revendications pour l'(les) Etat(s) contractant(s) suivant(s): ES)

1. Procédé pour préparer un traceur comprenant l'étape de couplage d'un précurseur de formule : 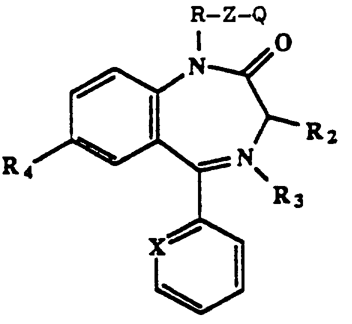
dans laquelle :
X est CH, N ou C-halogène,
R₂ est -H ou -OH,
R₃ est -O ou une paire d'électrons non liants,
R₄ est halogène, -NO₂, -NH₂ ou -NHCOCH₃,
R est un groupe de liaison consistant en 0 à 20 atomes de carbone et hétéroatomes, ne comprenant pas plus de 12 hétéroatomes, disposé en une chaîne linéaire ou ramifiée et contenant jusqu'à deux structures cycliques, à condition que pas plus de quatre hétéroatomes puissent être liés successivement et que pas plus de deux atomes de soufre ou d'azote ou un atome d'oxygène puissent être liés successivement,
Z est NH, CO, SO₂ ou -C=NH, et
Q est -H, -OH ou un groupe partant,
avec la fluorescéine ou un dérivé de la fluorescéine.

2. Procédé selon la revendication 1 dans lequel, lorsque ZQ est NH₂, le précurseur est couplé par les étapes de :
(a) préparation d'un ester actif de carboxyfluorescéine,
(b) réaction de l'ester actif avec le précurseur.

3. Procédé selon la revendication 1 dans lequel, lorsque ZQ est CO₂H, le précurseur est couplé par les étapes de :
(a) préparation d'un ester actif du précurseur,
(b) réaction de l'ester actif avec l'aminofluorescéine.

4. Procédé selon la revendication 1 dans lequel ZQ est NH₂ et le précurseur est mis à réagir avec la (4,6-dichloro-1,3,5-triazin-2-ylamino)fluorescéine ou l'isothiocyanate de fluorescéine.

5. Procédé selon la revendication 1 dans lequel un précurseur est couplé par les étapes de :
(a) préparation d'un ester imidate du précurseur,
(b) réaction de l'imidate avec l'aminofluorescéine.

6. Procédé selon la revendication 1 dans lequel, lorsque Q est OH, le précurseur est couplé par réaction du précurseur avec la (4,6-dichloro-1,3,5-triazin-2-ylamino)fluorescéine.

7. Procédé pour détecter la présence de benzodiazépines et de métabolites de benzodiazépines dans un échantillon de liquide qui comprend les étapes de :
(a) mise en contact dudit échantillon avec un antisérum produit contre une benzodiazépine liée à une substance support immunogène par son azote cyclique adjacent à son carbonyle cyclique et avec un composé capable de produire une réponse de polarisation de fluorescence détectable à la présence dudit antisérum, ledit composé comprenant la structure : 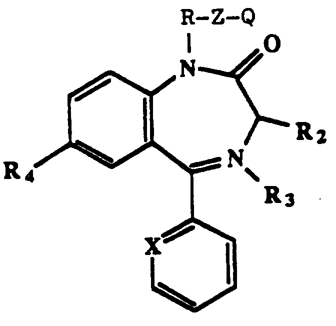
dans laquelle:
X est CH, N ou C-halogène,
R₂ est -H ou -OH,
R₃ est -O ou une paire d'électrons non liants,
R₄ est halogène, -NO₂, -NH₂ ou -NHCOCH₃,
R est un groupe de liaison consistant en 0 à 20 atomes de carbone et hétéroatomes, ne comprenant pas plus de 12 hétéroatomes, disposé en une chaîne linéaire ou ramifiée et contenant jusqu'à deux structures cycliques, à condition que pas plus de quatre hétéroatomes puissent être liés successivement et que pas plus de deux atomes de soufre ou d'azote ou un atome d'oxygène puissent être liés successivement,
Z est C=O, C=NH, NH, NCH₃, -N=N-, SO₂ ou CH₂, et
Q est un dérivé amino, amido, amidino, urée, thiourée, carbamate, thiocarbamate, triazinylamino ou (carboxyamino)-sulfonamido de fluorescéine ou Q est 4-chloro-6-fluorescéinylamino-1,3,5-triazin-2-yle ou 4-méthoxy-6-fluorescéinylamino-1,3,5-triazin-2-yle,
(b) passage d'une lumière polarisée dans un plan dans la solution résultante provenant de l'étape (a) pour obtenir une réponse de polarisation de fluorescence, et
(c) détection de la réponse de polarisation de fluorescence de la solution de l'étape (b) en tant que mesure de la présence de benzodiazépines et de métabolites de benzodiazépines dans l'échantillon.

8. Procédé selon la revendication 7 dans lequel Q est un dérivé triazinylamino de fluorescéine.

9. Procédé selon la revendication 7 dans lequel Q est 4-chloro-6-(fluorescéin-6-ylamino)-1,3,5-triazin-2-yle, fluorescéin-5-ylcarbonyle ou fluorescéin-6-ylcarbonyle.

10. Procédé selon la revendication 7 dans lequel Q est (fluorescéin-5-ylamino)carbonyle ou (fluorescéin-6-ylamino)carbonyle.

11. Procédé selon la revendication 7 dans lequel Q est (fluorescéin-5-yl)amino ou (fluorescéin-6-yl)amino.

12. Procédé selon la revendication 7 dans lequel l'antisérum est produit contre un immunogène comprenant la structure : 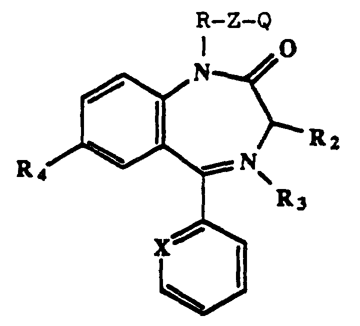
dans laquelle :
X est CH, N ou C-halogène,
R₂ est -H ou -OH,
R₃ est -O ou une paire d'électrons non liants,
R₄ est halogène, -NO₂, -NH₂ ou -NHCOCH₃,
R est un groupe de liaison consistant en 0 à 20 atomes de carbone et hétéroatomes, ne comprenant pas plus de 12 hétéroatomes, disposé en une chaîne linéaire ou ramifiée et contenant jusqu'à deux structures cycliques, à condition que pas plus de quatre hétéroatomes puissent être liés successivement et que pas plus de deux atomes de soufre ou d'azote ou un atome d'oxygène puissent être liés successivement,
Z est C=O, C=NH, NH, NCH₃, -N=N-, SO₂ ou CH₂, et
Q est une substance support immunogène.

13. Procédé selon la revendication 12 dans lequel, dans la formule dudit immunogène, R₄ est Cl et Z est C=O, C=NH, SO₂, NH, NCH₃ ou CH₂.

14. Procédé selon la revendication 12 dans lequel l'antisérum est produit contre le conjugué de 1-carboxyméthyl-7-chloro-1,3-dihydro-5-phényl-2H-1,4-benzodiazépin-2-one et de sérumalbumine bovine.

15. Procédé selon la revendication 14 dans lequel l'antisérum est produit chez le mouton ou le lapin.

16. Procédé selon la revendication 14 dans lequel l'antisérum a été produit chez le mouton et l'échantillon est de l'urine humaine.

17. Procédé selon la revendication 14 dans lequel le composé selon la revendication 12 est un mélange de deux composés dans lesquels R₂ est l'hydrogène, R₃ n'est pas O, R₄ est Cl, X est CH, Z est NH, Q est 4-chloro-6-(fluorescéin-6-ylamino)-1,3,5-triazin-2-yle et R est -CH₂CH₂- ou -CH₂CONHCH₂CH₂-.

18. Procédé de préparation d'un anticorps comprenant les étapes d'administration du conjugué de 1-carboxyméthyl-7-chloro-1,3-dihydro-5-phényl-2H-1,4-benzodiazépin-2-one et de sérumalbumine bovine à des animaux non humains ou à des cultures in vitro de cellules immunocompétentes par une série d'inoculations, lesdites inoculations déclenchant la production desdits anticorps.

19. Procédé selon la revendication 18 dans lequel le conjugué a été administré à des moutons ou des lapins.
